# EUROPÄISCHE PATENTSCHRIFT

(11) **EP 0 797 580 B1**
(45) Veröffentlichungstag und Bekanntmachung des Hinweises auf die Patenterteilung: **10.04.2002**
(21) Anmeldenummer: 95942675.0
(22) Anmeldetag: 15.12.1995
(51) Int. Cl.: C07H 19/02

(54) **NUCLEOSID-DERIVATE MIT PHOTOLABILEN SCHUTZGRUPPEN**
NUCLEOSIDE DERIVATIVES WITH PHOTOLABILE PROTECTIVE GROUPS
DERIVES DE NUCLEOSIDES COMPORTANT DES GROUPES PROTECTEURS PHOTOLABILES

(30) Priorität: 16.12.1994 DE 4444996
(43) Veröffentlichungstag der Anmeldung: 01.10.1997
(73) Patentinhaber: Pfleiderer, Wolfgang, 78464 Konstanz (DE)
(72) Erfinder: PFLEIDERER, Wolfgang, D-78464 Konstanz (DE); GIEGRICH, Heiner, D-78464 Konstanz (DE)
(74) Vertreter: Hansen, Bernd, Dr. Dipl.-Chem.
(86) Internationale Anmeldenummer: EP9504976
(87) Internationale Veröffentlichungsnummer: WO9618634

(56) Entgegenhaltungen:
- WO-A-94/10128
- PROC. NATL. ACAD. SCI. USA, Bd. 91, 1.Mai 1994, Seiten 5022-6, XP002003499 A.C. PEASE ET. AL.: "Light-generated oligonucleotide arrays for rapid DNA sequence analysis"
- W. PFLEIDERER ET AL.: "New Protecting Groups In Nucleoside and Nucleotide Chemistry" in: "Biophosphates and Their Analogues - Synthesis, Structure, Metabolism and Activity" 1987 , K.S. BRUZIK UND W.J. STEC XP002003501 in der Anmeldung erwähnt siehe Seite 133 - Seite 142
- J. ORG. CHEM., Bd. 60, 10.März 1995, Seiten 1116-7, XP002003500 M.C. PIRRUNG UND J-C. BRADLEY: "Dimethoxybenzoin Carbonates: Photochemically-Removable Alcohol Protecting Groups Suitable for Phosphoramidite-Based DNA Synthesis"

## Beschreibung

Gegenstand der vorliegenden Erfindung sind Nucleosid-Derivate mit photolabilen Schutzgruppen und Verfahren zu deren Herstellung.

Photolabile Schutzgruppen für die Hydroxy- und Phosphatfunktionen in Nucleosiden bzw. Nucleotiden sind von besonderem Interesse, da sie bspw. für lichtgesteuerte Parallel-Synthesen von Oligonucleotiden auf einem festen Träger geeignet sind (vgl. S.P.A. Fodor et al. Science 1991, 251, S.767ff). Mit ihrer Hilfe können sogenannte DNA-Chips (d.h. Trägerplättchen, auf deren Oberfläche viele verschiedene Oligonucleotide angeordnet sind) hergestellt werden, die wiederum in der Molekularbiologie für eine schnelle DNA-Sequenz-Analyse benötigt werden.

Entsprechend dem Stand der Technik wurden bisher als photolabile Schutzgruppen in der Nucleosid- bzw. Nucleotidchemie vor allem die o-Nitrobenzyl-Gruppe und ihre Derivate eingesetzt (vgl. V.N.R. Pillai, Org. Photochem. 1987, 9, S.225ff bzw. J.W. Walker et al., J. Am. Chem. Soc. 1988, 110, S.7170ff). Als besonders nachteilig bei diesen Schutzgruppen hat sich die langsame und zum Teil nur unvollständige Entschützung der entsprechenden Nucleosidbzw. Nucleotid-Derivate erwiesen. Außerdem entstehen bei der Abspaltung der o-Nitrobenzyl-Verbindungen z.T. unerwünschte Nebenprodukte in Form von toxischen Nitrosophenylverbindungen.

Als weitere photolabile Schutzgruppe für Nucleoside wurde entsprechend dem Artikel von W. Pfleiderer et al. in "Biophosphates and Their Analogues - Synthesis, Structure, Metabolism and Activity", Elsevier Science Publishers B.V. (Amsterdam) 1987, S.133ff die 2-(o-Nitrophenyl)ethylgruppe empfohlen, die jedoch ausschließlich als Schutzgruppe im Basenteil, insbesondere in O⁶-Stellung des Guanosins, eingeführt wird. In derselben Publikation werden auch die p-Nitrophenylethoxycarbonyl(NPEOC)- und die 2,4-Dinitrophenylethoxycarbonyl(DNPEOC)-Gruppen sowohl als Schutzgruppen für die Aminofunktion als auch für die Hydroxylfunktionen im Zuckerteil beschrieben, doch erfolgt die Abspaltung dieser Gruppen ausschließlich durch basenkatalysierte β-Eliminierung.

WO 94/10128 offenbart Nucleoside bzw. Nucleotide mit photolabilen 1-(Ortho-nitroaryl)-ethoxycarbonyl-Schutzgruppen. Diese werden zu lichtgesteuerten parallelen Oligonnucleotidesynthese an festen Trägern eingesetzt. Ähnliche Gruppen dieser Art sind auch in Proc. Natl. Acad. Sci. USA, 91, 5022-6 (1994) offenbart.

Der vorliegenden Erfindung lag die Aufgabe zugrunde, Nucleosid-Derivate mit photolabilen Schutzgruppen für die 5'-OH-Funktion im Zuckerteil zu entwickeln, welche die genannten Nachteile entsprechend dem Stand der Technik nicht aufweisen, sondern sich vergleichsweise schnell, quantitativ und ohne Bildung unerwünschter Nebenprodukte entschützen lassen.

Diese Aufgabe wurde erfindungsgemäß durch Nucleosid-Derivate der allgemeinen Formel (I) entsprechend Anspruch 1 gelöst. Es hat sich nämlich überraschenderweise gezeigt, daß sich die erfindungsgemäßen Schutzgruppen wesentlich schneller und vollständiger abspalten lassen als z.B. die o-Nitrobenzylgruppen. Außerdem konnten bei der Entschützung bisher keine unerwünschten Nebenprodukte festgestellt werden, was ebenfalls nicht vorhersehbar war.

Die erfindungsgemäßen Nucleosid-Derivate weisen folgende allgemeine Formel (I) auf: 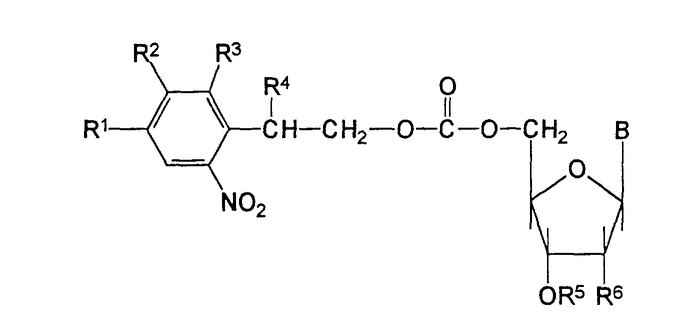 wobei die Reste R¹, R² und R³ am Phenylring folgende Bedeutung haben können:
- R¹ =: H, NO₂, CN, OCH₃, Halogen oder Alkyl oder Alkoxyalkyl mit 1 bis 4 C-Atomen
- R² =: H, OCH₃
- R³ =: H, F, Cl, Br, NO₂

Gemäß einer bevorzugten Ausführungsform bedeutet R³ = H, falls R² = OCH₃ ist.

Der Rest R⁴, der am C₂-Atom der o-Nitrophenylethyl-Gruppierung sitzt, kann entweder H, Halogen, OCH₃ oder ein Alkylrest mit 1 bis 4 C-Atomen sein. Der Alkylrest kann hierbei linear oder verzweigt, substituiert (insbesondere mit einem oder mehreren Halogenatomen) oder unsubstituiert sowie gesättigt oder ungesättigt sein; das gleiche gilt auch für die Alkyl- und Alkoxyalkylreste bei R¹. Vorzugsweise stellt R⁴ einen Methylrest dar. Im Falle von R⁴ ≠ H sind die Substituenten R¹, R² und R³ am Phenylring vorzugsweise Wasserstoffreste. Außerdem stellt im Falle von R² = OCH₃ R³ insbesondere einen Wasserstoffrest dar.

Halogen bedeutet in dieser Anmeldung durchwegs F, Cl, Br, I und vorzugsweise F, Cl oder Br.

Der Nucleosidteil der erfindungsgemäßen Verbindungen besteht aus den üblichen D-Ribofuranose- bzw. 2'-Desoxyribofuranose-Einheiten sowie den Pyrimidin- (B = Cytosin, Thymin, Uracil) bzw. Purinbasen (B = Adenin, Guanin). Als Basen können auch 2,6-Diaminopurin-9-yl-, Hypoxanthin-9-yl-, 5-Methylcytosin-1-yl-, 5-Amino-4-imidazolcarbonsäureamid-1-yl- oder 5-Amino-4-imidazolcarbonsäureamid-3-yl-Reste eingesetzt werden.

Die OH-Gruppe(n) im Ribofuranosid- bzw.
2'-Desoxyribofuranose-Teil können je nach Bedarf frei oder geschützt sein. Zum Schutz der 3'-Stellung haben sich hierbei die bekannten Phosphitamid-Gruppen bewährt, wie z.B. 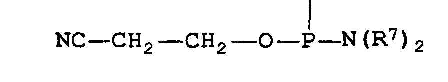 oder 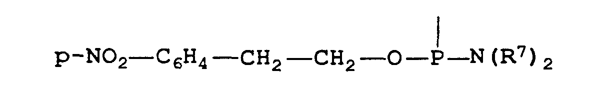 wobei die R⁷-Gruppen gleich oder verschieden sein können und lineare oder verzweigte Alkylreste mit 1 bis 4 C-Atomen bedeuten. Vorzugsweise sind sie Ethyl- oder Isopropylreste.

In der 2'-Stellung des Ribofuranosid-Teiles (Position R⁶) kann neben einem Wasserstoff- oder Halogenatom (insbesondere F, Cl, Br) eine freie oder geschützte OH-Gruppe vorliegen, wobei eine beliebige in der Nucleotidchemie übliche Schutzgruppe (R⁸) verwendet werden kann. Insbesondere kann auf die üblichen Alkyl-, Alkenyl-, Acetal- oder SilyletherSchutzgruppen für Sauerstoffatome (X=O) zurückgegriffen werden. R⁶ kann auch eine S-Alkylgruppe darstellen (X=S, R⁸=Alkyl). Bevorzugte Beispiele für O-Alkyl-Schutzgruppen sind O-Methyl- oder O-Ethylreste, für O-Alkenyl-Schutzgruppen O-Allylreste, für O-Acetal-Schutzgruppen O-Tetrahydropyranylbzw. O-Methoxytetrahydropyranyl-Reste sowie für O-Silylether-Schutzgruppen O-t-Butyldimethylsilyl-Reste.

Gemäß einer bevorzugten Ausführungsform können die Pyrimidinbzw. Purinbasen mit primären Aminofunktionen (z.B. Adenin, Cytosin und Guanin) noch permanente Schutzgruppen vorzugsweise auf Carbonylbasis aufweisen. Bevorzugt sind hierbei vor allem Phenoxyacetyl- oder Dimethylformamidino-Reste, die für alle drei genannten Basen in Frage kommen. Daneben gibt es noch spezielle Schutzgruppen, die nur bei bestimmten Basen eingeführt werden. Dies sind bspw. im Falle von Adenin Benzoyl- oder p-Nitrophenylethoxycarbonyl (p-NPEOC)-Reste. Für Guanin können neben den p-NPEOC-Resten auch Isobutyroyl-Schutzgruppen eingeführt werden. Schließlich eignen sich für Cytosin neben den p-NPEOC-Resten auch noch Benzoyl-Schutzgruppen.

Die Herstellung der erfindungsgemäßen Nucleosid-Derivate kann in zwei Stufen erfolgen. In der ersten Stufe a) wird ein Alkohol der allgemeinen Formel (II) 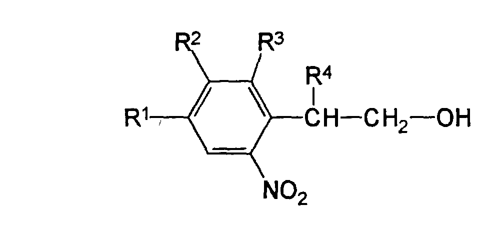 in der R¹, R², R³, R⁴ die oben angegebene Bedeutung besitzen, mit einem Phosgen-Derivat vorzugsweise in einem unpolaren organischen Lösemittel bei Temperaturen zwischen -20 und +25°C zur Umsetzung gebracht. Als Phosgen-Derivat kann neben dem bevorzugten Phosgen auch Diphosgen (Chlorameisensäuretrichlormethylester) oder Triphosgen (Bistrichlormethylcarbonat) verwendet werden.

Die Alkoholkomponente ist in den meisten Fällen bekannt oder kann in analoger Weise nach bekannten Verfahren hergestellt werden. Als unpolares organisches Lösemittel wird in Stufe a) vorzugsweise Toluol oder THF verwendet. Die Reaktionskomponenten können zwar in annähernd stöchiometrischem Verhältnis eingesetzt werden, doch wird das Phosgen-Derivat vorzugsweise in deutlichem Überschuß, bspw. in zwei- bis fünffachem molaren Überschuß, bezogen auf die Alkoholkomponente eingesetzt. Auch die Konzentration der Alkoholkomponente kann in weiten Grenzen variiert werden, doch hat es sich als besonders vorteilhaft erwiesen, diese Konzentration auf 0,1 bis 10,0 mmol pro 10 ml Solvens einzustellen.

Bei dieser Reaktion (Reaktionsdauer ca. 1 bis 6 Std.) entstehen in guter Reinheit und hoher Ausbeute (> 90 %) die entsprechenden Chlorkohlensäureester der allgemeinen Formel (IV)

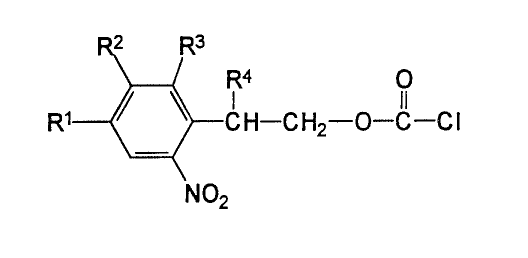

Die Aufarbeitung der entsprechenden Produkte erfolgt vorzugsweise, indem man zunächst das überschüssige Phosgen sowie das Lösemittel im Vakuum abdestilliert. Der Chlorkohlensäureester (IV) kann dann ohne weitere Aufarbeitung in Stufe b) mit den Nucleosiden der allgemeinen Formel (III) 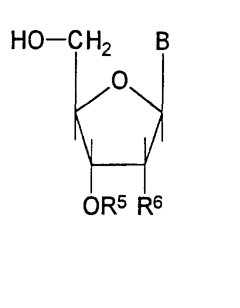 umgesetzt werden, wobei R⁵, R⁶ und B die oben angegebene Bedeutung besitzen.

Die Umsetzung erfolgt vorzugsweise in einem Lösemittelgemisch bestehend aus Dichlormethan und einem polaren organischen Lösemittel ggf. in Gegenwart einer Base bei Temperaturen zwischen -60 und +25°C. Als polares organisches Lösemittel wird hierbei bevorzugt DMF oder Pyridin eingesetzt, wobei im Falle von Pyridin keine zusätzliche Base erforderlich ist. Wird jedoch mit Dichlormethan/DMF-Lösemittelgemischen gearbeitet, empfiehlt sich die Zugabe einer Base wie z.B. Pyridin, Triethylamin oder Ethyldiisopropylamin, um die bei der Reaktion freigesetzten Protonen abzufangen. Das Mischungsverhältnis Dichlormethan zu Pyridin bzw. DMF ist ebenfalls unkritisch, doch werden vorzugsweise 1 bis 3 Vol.-teile Dichlormethan pro Vol.-teil Pyridin bzw. DMF eingesetzt.

Gemäß einer bevorzugten Ausführungsform wird das entsprechende Nucleosid (III), welches in Pyridin oder DMF/Base gelöst wurde, vorgelegt und eine Lösung des Chlorkohlensäureesters in Dichlormethan bei der jeweiligen Reaktionstemperatur zugetropft. Das Molverhältnis von Nucleosid zu Chlorkohlensäureester kann hierbei entsprechend der Stöchiometrie auf ca. 1:1 eingestellt werden. vorzugsweise wird jedoch der Chlorkohlensäureester im Überschuß eingesetzt, und zwar in einer solchen Menge, daß das Molverhältnis Nucleosid zu Chlorkohlensäureester 1:1 bis 1:2 beträgt. Schließlich kann auch die Konzentration des Nucleosids im Lösemittelgemisch in weiten Grenzen variiert werden, doch wird es bevorzugt auf 0,1 bis 3,0 mmol pro 10 ml Solvens eingestellt.

Nach erfolgter Umsetzung (Reaktionszeit ca. 1 bis 5 Std.) können die erfindungsgemäßen Nucleosid-Derivate nach bekannten Methoden isoliert bzw. gereinigt werden, wie z.B.

Verdünnen mit Dichlormethan, Auswaschen aller Salze mit Wasser, Trocknen der organischen Phase, Einengen der Lösung bzw. Kristallisation und anschließende Kieselgel-Chromatographie. Auf diese Weise können die entsprechenden Nucleosid-Derivate mit hoher Reinheit und in guten Ausbeuten (60 bis 85 %) erhalten werden.

Gemäß einer bevorzugten Ausführungsform kann man im Anschluß an die Reaktionsstufe b) in die 3'-Stellung der Nucleosid-Derivate mit R⁵ = H die Phosphitamid-Gruppe 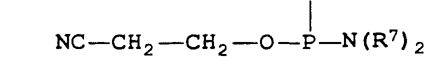 oder 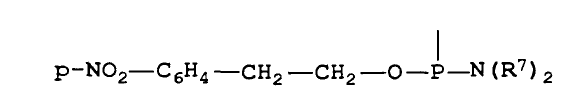 nach bekannten Methoden einführen. Üblicherweise erfolgt diese Umsetzung mit den entsprechenden Phosphinen in Gegenwart von 1H-Tetrazol als Aktivator in einem Lösemittelgemisch bestehend aus Dichlormethan und Acetonitril bei Temperaturen zwischen 0 und 25°C. Vorzugsweise wird das Phosphin in zwei- bis dreifachem molaren Überschuß eingesetzt, während das Molverhältnis Phosphin zu 1H-Tetrazol auf 3:ca.1,0 eingestellt wird. Das Mengenverhältnis von Dichlormethan zu Acetonitril ist relativ unkritisch und beträgt vorzugsweise 1:1 bis 4:1. Nach erfolgter Umsetzung (ca. 10 bis 20 h) kann das entsprechende Nucleosid wie in Stufe b) beschrieben aufgearbeitet werden.

Wie Bestrahlungsversuche mit polychromatischem Licht mit Wellenlänge > 289 nm belegen, lassen sich die erfindungsgemäßen Nucleoside sehr rasch (t_{0,5} = 1 bis 7 Min.) und weitgehend entschützen (Ausbeuten bis zu 97 %), sodaß die besonderen Anforderungen an die Photolabilität der Schutzgruppen in hervorragender Weise erfüllt werden.

Aufgrund dieser besonderen Eigenschaften eignen sich die erfindungsgemäßen Nucleoside sehr gut für die Herstellung von Oligonucleotiden durch lichtgesteuerte Schutzgruppenabspaltung insbesondere auf festen Trägerplatten.

Die nachfolgenden Beispiele sollen die Erfindung näher erläutern.

### Beispiel 1

### a) 2-(2-Nitrophenyl)ethanol [1, 2]

Ein Gemisch von o-Nitrotoluol (9,2 g, 67 mmol) und Paraformaldehyd (0,8 g, 25 mmol) in DMSO (10 ml, Synthese-Qualität, zusätzlich 2 d über Molekularsieb 4 Å getrocknet) wurde mit KOH (21 mg, 0,37 mmol) versetzt und 2,5 h bei 95°C gerührt. Das Lösemittel wurde im Hochvakuum abdestilliert. Der Rückstand wurde durch Säulenchromatographie (SiO₂, 20 x 3 cm, Lösemittel: Toluol 750 ml, Toluol/EtOAc 10:1 500 ml, 7:1 500 ml, 5:1 500 ml) gereinigt. Man erhielt 2-(2-Nitrophenyl)ethanol (2,33 g, 21 %) als gelbes Öl.
R_{f} (SiO₂, Toluol/EtOAc 10:1) 0,21
UV(MeOH), λₘₐₓ [nm] (log ε): 205 (4,07), 256 (3,65), 348 (Schulter, 2,56)
¹H-NMR (250 MHz, CDCl₃): 7,93 (dd, 1 arom. H, ortho zu NO₂); 7,49 (m, 3 arom. H); 3,96 (t, α-CH₂); 3,17 (t, β-CH₂), 1,72 (s, OH)
C₈H₉NO₃ (167,2)

### Literatur:

[1] G. M. Bennet, M. M. Hafez, J. Chem. Soc. 1941, 287
[2] E. Uhlmann, W. Pfleiderer, Helv. Chim. Acta 1981, 64, 1688

### b) 2-(2-Nitrophenyl)ethoxycarbonylchlorid

In eine Lösung von 2-(2-Nitrophenyl)ethanol (5,2 g, 31 mmol) in THF (20 ml, dest. über CaH₂) wurde bei Raumtemperatur unter Rühren Phosgen eingeleitet. Nach 1,5 h wurde das überschüssige Phosgen sowie das Lösemittel im Hochvakuum abdestilliert. Man erhielt 2-(2-Nitrophenyl)ethoxycarbonylchlorid (6,69 g, 94 %) als gelbes Öl.
R_{f} (SiO₂, CHCl₃) 0,84
UV(CH₃CN), λₘₐₓ [nm] (log ε) : 202 (4,12), 218 (Schulter, 3,74); 256 (3,70); 298 (Schulter, 3,16); 346 (Schulter, 2,59)
¹H-NMR (250 MHz, CDCl₃): 7,99 (dd, H-C(3)); 7,48 (m, 3 arom. H); 4,62 (t, α-CH₂); 3,31 (t, β-CH₂)
Anal. ber. für C₉H₈ClNO₄ (229,62): C 47,08, H 3,51, N 6,10; gef.: C 47,30, H 3,70, N 6,00

### c) 5'-O- (2-(2-Nitrophenyl)ethoxycarbonyl)thymidin

Thymidin (1 g, 4,13 mmol) wurde mit Pyridin koevaporiert (2 x 10 ml pro analysi-Qualität, zusätzlich über Molekularsieb 4 Å getrocknet), in Pyridin (10 ml, s.o.) gelöst und auf -30°C gekühlt. In 10 min wurde hierzu eine Lösung von 2-(2-Nitrophenyl)-ethoxycarbonylchlorid (1,45 g, 6,31 mmol) getropft. Nach weiteren 4 h 50 min Rühren unter i-PrOH/N₂-Kühlung (-30 bis -15°C) wurde das Gemisch mit CH₂Cl₂ (150 ml) verdünnt und mit H₂O (150 ml) gewaschen. Die wäßrigen Phasen wurden mit CH₂Cl₂ (2 x 150 ml) nachextrahiert. Die vereinigten organischen Phasen wurden über MgSO₄ getrocknet, filtriert, einrotiert und mit Toluol (5 x 20 ml) und CH₂Cl₂ (2 x 20 ml) koevaporiert. Das Rohprodukt wurde durch Säulenchromatographie (SiO₂, 15 x 3,5 cm, Lösemittel: CH₂Cl₂ 1300 ml, CH₂Cl₂/Aceton 20:1 1200 ml, 10:1 600 ml, 8:1 500 ml, 5:1 500 ml, 4:1 500 ml, 2:1 750 ml, 1:1 500 ml) gereinigt. Man erhielt 5'-O-(2-(2-Nitrophenyl)ethoxycarbonyl)thymidin (1,15 g, 64 %) als farblosen Feststoff.
R_{f} (SiO₂, Toluol/EtOAc/MeOH 5:4:1) 0,46
UV(MeOH), λₘₐₓ [nm] (log ε): 205 (4,31), 262 (4,12), 334 (Schulter, 2,64)
¹H-NMR (250 MHz, CDCl₃): 8,92 (s (br), NH); 7,96 (dd, (H-C(3) v. NPEOC); 7,55 (t, 1 arom. H v. NPEOC); 7,42 (m, H-C(6) v. Thymin, 2 arom. H v. NPEOC); 6,35 (t, H-C(1')); 4,44 (m, H-C(3'), 2 x H-C(5'), α-CH₂ v. NPEOC); 4,15 (q, H-C(4'); 3,34 (m, β-CH₂ v. NPEOC); 2,89 (d, OH-C(3')); 2,41 (m, H-C(2')); 2,22 (m, H-C(2')); 1,85 (s, CH₃)
Anal. ber. für C₁₉H₂₁N₃O₉ (435,39): C 52,41, H 4,86, N 9,65; gef.: C 52,07, H 5,15, N 9,65

### Beispiel 2

### a) 2-(2, 6-Dinitrophenyl)ethanol [1]

Zu 2,6-Dinitrotoluol (18,2 g, 0,1 mol, 3 d im Hochvakuum über Blaugel getrocknet) und Paraformaldehyd (3 g, 0,1 mol) in DMSO (50 ml, Synthese-Qualität, zusätzlich 2 d über Molekularsieb 4 Å getrocknet) wurde eine Lösung von Kaliumtertiärbutylat (1,8 g, 8 mmol) in tert.-Butanol (20 ml, Synthese-Qualität, 99 %) gegeben. Nach der Zugabe der Kaliumtertiärbutylat-Lösung trat ein Farbumschlag von gelb nach tief-violett ein. Es wurde 5 min bei Raumtemperatur und 10 min bei 70°C (Ölbadtemperatur) gerührt. Danach ließ man auf Raumtemperatur abkühlen, neutralisierte das Gemisch mit konz. HCl, verdünnte mit H₂O (300 ml) und versetzte es mit NaCl bis zur Sättigung. Die wäßrige Phase wurde mit EtOAc (3 x 500 ml) extrahiert. Die vereinigten organischen Phasen wurden mit gesättigter NaCl Lösung (300 ml) gewaschen, über Na₂SO₄ getrocknet, filtriert und einrotiert. Das Rohprodukt (24,3 g) wurde in wenig EtOAc in der Siedehitze gelöst, mit Petrolether (nachfolgend PE genannt) (100 ml) versetzt und im Eisfach zur Kristallisation gebracht. Der Niederschlag wurde abgesaugt und durch Säulenchromatographie (20,7 g verunreinigtes Produkt, 300 g SiO₂, 18 x 6,5 cm, Lösemittel: Toluol/EtOAc 5:1, 4:1, 3:1) gereinigt. Mischfraktionen wurden einrotiert und durch erneute Säulenchromatographie (200 g SiO₂, 20 x 5,3 cm, Lösemittel: Toluol/EtOAc 7:1) gereinigt. Man erhielt nach Einrotieren der reinen Produktfraktionen 2-(2,6-Dinitrophenyl)ethanol (13,6 g, 64 %) als gelben Feststoff.
R_{f} (SiO₂, Toluol/EtOAc 9:1) 0,21
Schmelzpunkt: 69 bis 70°C (Lit. [1]: 69°C)
UV(MeOH), λₘₐₓ [nm] (log ε): 203 (4,20), 231 (4,00), 280 (Schulter, 3,17), 327 (Schulter, 2,80)
¹H-NMR (250 MHz, CDCl₃): 8,00 (d, J = 8,1, H-C(3), H-C(5)); 7,57 (t, J = 8,1, H-C(4)); 3,97 (q, α-CH₂); 3,33 (t, β-CH₂); 1,69 (t, OH)
Anal. ber. für C₈H₈N₂O₅ (212,16): C 45,29, H 3,80, N 13,20; gef.: C 45,39, H 3,90, N 13,32

### Literatur:

[1] N. S. Girgis, H. B. Cottam, R. K. Robins, J. Heterocycl. Chem. 1988, 25, 361

### b) 2-(2,6-Dinitrophenyl)ethoxycarbonylchlorid

Zu einer auf 0°C gekühlten Lösung von Chlorameisensäuretrichlormethylester (3,81 g, 2,33 ml, 19,28 mmol) in THF (10 ml, dest. über CaH₂) wurde in 20 min eine Lösung von 2-(2,6-Dinitrophenyl)ethanol (4,08 g, 19,23 mmol) und Et₃N (2,7 ml, 19,28 mmol) in THF (30 ml, s.o.) zugetropft. Es wurde 25 min unter Eisbadkühlung und 1 h 45 min bei Raumtemperatur gerührt. Die Mischung wurde über Celite filtriert. Nachwaschen des Filterkuchens mit THF, Abdestillieren des Lösemittels sowie des überschüssigen Reagenses von den vereinigten Filtraten und Trocknen im Hochvakuum ergaben 5,13 g 2-(2,6-Dinitrophenyl)-ethoxycarbonylchlorid (97 %) als hellbraunen Feststoff.
Rf (SiO₂, CH₂Cl₂) 0,76
Schmelzpunkt: 84 bis 85°C
UV(CH₃CN), λₘₐₓ [nm] (log ε): 233 (4,02), 292 (Schulter, 3,11), 331 (Schulter, 2,82)
¹H-NMR (250 MHz, CDCl₃): 8,10 (d, J = 8,2, H-C(3), H-C(5)); 7,67 (t, J = 8,1, H-C(4)); 4,67 (t, α-CH₂); 3,50 (t, β-CH₂) ;
Anal. ber. für C₉H₇ClN₂O₆ (274,616): C 39,36, H 2,57, N 10,20; gef.: C 39,40, H 2,60, N 10,20

### c) 5'-O-(2-(2,6-Dinitrophenyl)ethoxycarbonyl)thymidin

Thymidin (1 g, 4,13 mmol) wurde mit Pyridin (3 x 10 ml, pro analysi-Qualität, zusätzlich über Molekularsieb 4 Å getrocknet) koevaporiert, in Pyridin (15 ml, s.o.) gelöst und auf -50°C gekühlt. Hierzu tropfte man in 1 h eine Lösung von 2-(2,6-Dinitrophenyl)ethoxycarbonylchlorid (1,7 g, 6,19 mmol) in CH₂Cl₂ (15 ml, dest. über CaH₂). Nach weiteren 3,5 h Rühren unter i PrOH/N₂-Kühlung (-50 bis -20°C) wurde das Gemisch mit CH₂Cl₂ (50 ml) verdünnt und mit H₂O (50 ml) gewaschen. Die wäßrigen Phasen wurden mit CH₂Cl₂ (2 x 50 ml) nachextrahiert. Die vereinigten organischen Phasen wurden über Na₂SO₄ getrocknet, filtriert, einrotiert und mit Toluol (3 x 50 ml) koevaporiert. Das Rohprodukt (2,66 g) wurde in CH₂Cl₂/MeOH 2:1 (60 ml) in der Siedehitze digeriert. Der erhaltene weiße Niederschlag wurde abgesaugt und aus MeOH (25 ml) umkristallisiert. Man erhielt 5'-O-(2-(2,6-Dinitrophenyl)ethoxycarbonyl)thymidin (855 mg, 43 %) als farblosen Feststoff. Die vereinigten Filtrate wurden zur Trockene einrotiert und durch Säulenchromatographie (1,4 g Rohprodukt, 56 g SiO₂ 17 x 3 cm, CH₂Cl₂/MeOH 100:5 1240 ml, 100:7 105 ml, 100:10 330 ml) gereinigt. Man erhielt 5'-O-(2-(2, 6-Dinitrophenyl)ethoxycarbonyl)thymidin (691 mg, 34,8 %) als farblosen Feststoff. Die Ausbeute an 5'-O-(2-(2,6-Dinitrophenyl)ethoxycarbonyl)thymidin betrug insgesamt 1,55 g (78 %).
R_{f} (SiO₂, Toluol/EtOAc/MeOH 5:4:1) 0,41
UV(MeOH), λₘₐₓ [nm] (log ε): 206 (4,28), 244 (Schulter, 4,07), 256 (4,08) 355 (Schulter, 2,72)
¹H-NMR (250 MHz, DMSO-d⁶): 11,32 (s, NH), 8,27 (d, H-C(3), H-C(5)); 7,78 (t, H-C(4)); 7,40 (s, H-C(6) v. Thymin); 6,18 (t, H-C(1')); 5,44 (d, OH-C(3')); 4,37 (t, α-CH₂); 4,23 (m, H-C(3'), 2 x H-C(5')); 3,91 (m, H-C(4')); 3,29 (t, β-CH₂); 2,15 (m, 2 x H-C(2')); 1,71 (s, CH₃)
Anal. ber. für C₁₉H₂₀N₄O₁₁ (480,386): C 47,51, H 4,20, N 11,66; gef.: C 47,40, H 4,15, N 11,57

### Beispiel 3

### a) 2-(2-Fluor-6-nitrophenyl)ethanol [1]

Zu 2-Fluor-6-nitrotoluol (776 mg, 5 mmol) und Paraformaldehyd (150 mg, 5 mmol) in DMSO (2,5 ml, Synthese-Qualität, zusätzlich 2 d über Molekularsieb 4 Å getrocknet) wurde eine Lösung von Kaliumtertiärbutylat (90 mg, 0,8 mmol) in tert.-Butanol (1 ml, Synthese-Qualität, 99 %) gegeben. Nach der Zugabe der Kaliumtertiärbutylat-Lösung trat ein Farbumschlag von gelb nach tief-violett ein. Es wurde 5 min bei Raumtemperatur und 30 min bei 70°C (Ölbadtemperatur) gerührt. Danach ließ man auf Raumtemperatur abkühlen und neutralisierte mit wenigen Tropfen konz. HCl. Das Gemisch wurde mit EtOAc (30 ml) verdünnt und mit H₂O (20 ml) gewaschen. Die wäßrige Phase wurde mit EtOAc (2 x 20 ml) nachextrahiert. Trocknen der organischen Phasen über Na₂SO₄, Filtrieren und Abrotieren des Lösemittels ergaben das Rohprodukt (1,11 g), welches durch Säulenchromatographie (20 g SiO₂, 12 x 2 cm, Lösemittel: PE 40 ml, PE/EtOAc 10:1 110 ml, 8:1 270 ml, 6:1 210 ml, 5:1 60 ml, 4:1 50 ml) gereinigt wurde. Man erhielt 2-(2-Fluor-6-nitrophenyl)ethanol (653 mg, 71 %) als gelben Feststoff.
R_{f} (SiO₂, Toluol/EtOAc 9:1) 0,24
UV(MeOH), λₘₐₓ [nm] (log ε): 205 (4,06), 251 (3,61), 294 (Schulter, 3,21)
¹H-NMR (250 MHz, CDCl₃): 7,73 (m, 1 arom. H); 7,36 (m, 2 arom. H); 3,94 (t, α-CH₂); 3,21 (dt, J = 2,2, 6,5, β-CH₂); 1,67 (s (br), OH)
Anal. ber. für C₈H₈FNO₃ (185,154): C 51,90, H 4,36, N 7,57; gef.: C 51,92, H 4,40, N 7,42

### Literatur:

[1] Chem. Abstr. 1989, 110, P 75032 k

### b) 2-(2-Fluor-6-nitrophenyl)ethoxycarbonylchlorid

Zu einer auf 0°C gekühlten Lösung von Chlorameisensäuretrichlormethylester (641 mg, 3,24 mmol) in THF (6,75 ml, dest. über CaH₂) wurde in 5 min eine Lösung von 2-(2-Fluor-6-nitrophenyl)ethanol (500 mg, 2,7 mmol) und Et₃N (273 mg, 2,7 mmol, dest. über KOH) in THF (6,75 ml, s.o.) zugegeben. Man ließ 1 h unter Eisbadkühlung und 1 h bei Raumtemperatur rühren. Das Gemisch wurde über Celite filtriert. Nachwaschen des Filterkuchens mit THF und Abdestillieren des Lösemittels sowie des überschüssigen Reagenses von den vereinigten Filtraten bei 30°C im Hochvakuum ergaben 2-(2-Fluor-6-nitrophenyl)ethoxycarbonylchlorid (620 mg, 93 %) als hellbraunes Öl.
R_{f} (SiO₂, PE/EtOAc 19:1) 0,25
UV(MeOH), λₘₐₓ [nm] (log ε): 204 (4,04), 251 (3,67), 293 (Schulter, 3,23)
¹H-NMR (250 MHz, CDCl₃): 7,83 (d, J = 7,7, 1 arom. H); 7,44 (m, 2 arom. H); 4,63 (t, α-CH₂); 3,37 (dt, J = 1,6, 6,4 β-CH₂)
Anal. ber. für C₉H₇ClFNO₄ (247,609): C 43,66, H 2,85, N 5,66; gef.: C 43,97, H 3,02, N 5,59

### c) 5'-O-(2-(2-Fluor-6-nitrophenyl)ethoxycarbonyl)thymidin

Thymidin (200 mg, 0,83 mmol) wurde mit Pyridin (3 x 3 ml, pro analysi-Qualität, zusätzlich über Molekularsieb 4 Å getrocknet) koevaporiert, in Pyridin (3 ml, s.o.) gelöst und auf -60°C (i-PrOH/N₂) gekühlt. Hierzu tropfte man in 20 min eine Lösung von 2-(2-Fluor-6-nitrophenyl)ethoxycarbonylchlorid (280 mg, 1,13 mmol) in CH₂Cl₂ (3 ml, dest. über CaH₂). Man ließ 3 h 40 min unter i-PrOH/N₂ Kühlung (-60 bis -15°C) und danach 1 h ohne Kältebad rühren, wobei die Temperatur gegen Ende bei 0°C lag. Die Reaktionsmischung wurde mit CH₂Cl₂ (10 ml) verdünnt und mit H₂O (10 ml) gewaschen. Die wäßrige Phase wurde mit CH₂Cl₂ (3 x 10 ml) nachextrahiert. Trocknen der organischen Phasen über Na₂SO₄, Filtrieren, Abrotieren des Lösemittels und Koevaporieren mit Toluol (3 x 10 ml) ergaben das Rohprodukt, welches durch Säulenchromatographie (20 g SiO₂ 12 x 2 cm, Lösemittel: CH₂Cl₂/MeOH 100:1 50 ml, 100:2 102 ml, 100:3 206 ml, 100:3,5 103 ml, 100:4 208 ml) gereinigt wurde. Man eluierte zuerst 3',5'-Bis-O-(2-(2-Fluor-6-nitrophenyl)ethoxycarbonyl)-thymidin, dann 3'-O-(2-(2-Fluor-6-nitrophenyl)-ethoxycarbonyl)thymidin und zuletzt 5'-O-(2-(2-Fluor-6-nitrophenyl)ethoxycarbonyl)thymidin. Nach Abrotieren des Lösemittels und Trocknen im Hochvakuum erhielt man 3',5'-Bis-O-(2-(2-Fluor-6-nitrophenyl)ethoxycarbonyl)thymidin (27 mg, 5 %) als hellgelben Schaum, 3'-O-(2-(2-Fluor-6-nitrophenyl)ethoxycarbonyl)thymidin (15 mg, 4 %) als farblosen Schaum und 5'-O-(2-(2-Fluor-6-nitrophenyl)-ethoxycarbonyl)thymidin (262 mg, 70 %) als farblosen Feststoff.
5'-O-(2-(2-Fluor-6-nitrophenyl)ethoxycarbonyl)thymidin:
R_{f} (SiO₂, Toluol/EtOAc/MeOH 5:4:1) 0,44
UV(MeOH), λₘₐₓ [nm] (log ε): 205 (4,29), 261 (4,10),
¹H-NMR (250 MHz, CDCl₃): 8,09 (s (br), NH); 7,76 (m, 1 arom. H v. FNPEOC); 7,41 (m, H-C(6) v. Thymin, 2 arom. H v. FNPEOC), 6,35 (t, H-C(1')); 4,45 (m, H-C(3'), 2 x H-C(5'), α-CH₂ v. FNPEOC); 4,14 (q, J = 3,2, H-C(4')); 3,36 (m, β-CH₂ v. FNPEOC); 2,40 (m, H-C(2')); 2,23 (m, H-C(2'), OH-C(3')); 1,85 (s, CH₃)
Anal. ber. für C₁₉H₂₀FN₃O₉ (453,379): C 50,34, H 4,45, N 9,27; gef.: C 50,22, H 4,49, N 9,18

### Beispiel 4

### a) 2-(2-Chlor-6-nitrophenyl)ethanol [1, 2]

Zu einem Gemisch aus 2-Chlor-6-nitrotoluol (25 g, 146 mmol) und Paraformaldehyd (1,9 g, 60 mmol) in DMSO (20 ml, Synthese-Qualität, zusätzlich 2 d über Molekularsieb 4 Å getrocknet) gab man Triton B (2 ml, 35 % in MeOH) und ließ bei 90°C rühren. Nach 2 h wurde das Reaktionsgemisch mit wenigen Tropfen konz. HCl neutralisiert, mit H₂O (50 ml) verdünnt und mit EtOAc (4 x 150 ml) extrahiert. Die vereinigten organischen Phasen wurden über MgSO₄ getrocknet, filtriert und einrotiert. Das Rohprodukt wurde durch Sublimation im Hochvakuum (p = 0,06 Torr, Ölbadtemperatur 95°C) gereinigt. Man erhielt 2-(2-Chlor-6-nitrophenyl)ethanol (8,01 g, 66 %) in Form von hellgelben Kristallen.
R_{f} (SiO₂, Toluol/EtOAc 10:1) 0,36
Schmelzpunkt: 59 bis 61°C
UV(MeOH), λₘₐₓ [nm] (log ε): 212 (4,13), 248 (3,48), 294 (Schulter, 3,03), 336 (Schulter, 2,58)
¹H-NMR (250 MHz, CDCl₃): 7,70 (dd, 1 arom. H); 7,62 (dd, 1 arom. H); 7,31 (t, H-C(4)); 3,94 (q, α-CH₂); 3,26 (t, β-CH₂); 1,70 (t, OH)
Anal. ber. für C₈H₈ClNO₃ (201,61): C 47,66, H 4,00, N 6,95; gef.: C 47,79, H 4,06, N 6,92

### Literatur:

[1] T. Morimoto, I. Hashimoto, H. Yamaoka, Chem. Abstr. 1978, 88, 104880 v.
[2] Y. Tsuji, S. Kotachi, K.-T. Huh, Y. Watanabe, J. Org. Chem. 1990, 55, 580

### b) 2-(2-Chlor-6-nitrophenyl)ethoxycarbonylchlorid

In eine Lösung von 2-(2-Chlor-6-nitrophenyl)ethanol (31 g, 154 mmol) in THF (190 ml, dest. über CaH2) wurde bei Raumtemperatur unter Rühren Phosgen eingeleitet. Nach 2,5 h wurden das überschüssige Phosgen sowie das Lösemittel im Hochvakuum abdestilliert. Man erhielt 2-(2-Chlor-6-nitrophenyl)ethoxycarbonylchlorid (39,4 g, 97 %) als gelbes Öl.
R_{f} (SiO₂, CHCl₃) 0,76
UV(CH₃CN), λₘₐₓ [nm] (log ε): 211 (4,14), 253 (3,53), 300 (Schulter, 3,02)
¹H-NMR (250 MHz, CDCl₃): 7,81 (dd, 1 arom. H); 7,69 (dd, 1 arom. H); 7,41 (t, H-C(4)); 4,63 (t, α-CH₂); 3,46 (t, β-CH₂)
Anal. ber. für C₉H₇Cl₂NO₄ (264,06): C 40,94, H 2,67, N 5,30; gef.: C 41,05, H 2,73, N 5,00

### c) 5'-O-(2-(2-Chlor-6-nitrophenyl)ethoxycarbonyl)thymidin

Thymidin (4 g, 16,5 mmol) wurde mit Pyridin (3 x 40 ml, pro analysi-Qualität, zusätzlich über Molekularsieb 4 Å getrocknet) koevaporiert, in Pyridin (48 ml, s.o.) gelöst und auf -50°C (i-PrOH/N₂) gekühlt. Hierzu tropfte man in 2 h 45 min eine Lösung von 2-(2-Chlor-6-nitrophenyl)-ethoxycarbonylchlorid (5,2 g, 19,8 mmol) in CH₂Cl₂ (48 ml, dest. über CaH₂). Man ließ weitere 30 min unter i-PrOH/N₂ Kühlung (-50 bis -30°C) rühren. Die Reaktionsmischung wurde mit CH₂Cl₂ (100 ml) verdünnt und mit H₂O (100 ml) gewaschen. Die wäßrige Phase wurde mit CH₂Cl₂ (2 x 100 ml) nachextrahiert. Trocknen der organischen Phasen über Na₂SO₄, Filtrieren, Abrotieren des Lösemittels und Koevaporieren mit Toluol (3 x 50 ml) ergaben das Rohprodukt, welches durch Säulenchromatographie (305 g SiO₂, 22 x 5,9 cm, Lösemittel: CH₂Cl₂ 200 ml, CH₂Cl₂/MeOH 100:2 1020 ml, 100:3 515 ml, 100:4 1560 ml, 100:5 850 ml, 100:6 318 ml, 100:8 324 ml, 100:9 654 ml) gereinigt wurde. Man eluierte zuerst eine Mischfraktion (1,4 g) von 3',5'-Bis-O-(2-(2-Chlor-6-nitrophenyl)ethoxycarbonyl)thymidin und 3'-0-(2-(2-Chlor-6-nitro-phenyl)ethoxycarbonyl)thymidin, dann eine Mischfraktion (367 mg) von 3'-O-(2-(2-Chlor-6-nitrophenyl)ethoxycarbonyl)-thymidin und 5'-0-(2-(2-Chlor-6-nitrophenyl)ethoxycarbonyl)-thymidin und zuletzt eine reine Fraktion von 5'-O-(2-(2-Chlor-6-nitrophenyl)ethoxycarbonyl)thymidin (6,21 g, 80 %, farbloser Feststoff). Die Mischfraktionen wurden durch weitere Säulenchromatographie gereinigt. Man erhielt 3',5'-Bis-O-(2-(2-Chlor-6-nitrophenyl)ethoxycarbonyl)thymidin (1,175 g, 10 %) als hellgelben Schaum, 3'-O-(2-(2-Chlor-6-nitrophenyl)ethoxycarbonyl)thymidin (203 mg, 3 %) als farblosen Schaum und 5'-O-(2-(2-Chlor-6-nitrophenyl)ethoxycarbonyl)thymidin (182 mg, 2 %) als farblosen Feststoff. Die Gesamtausbeute an 5'-O-(2-(2-Chlor-6-nitrophenyl)ethoxycarbonyl)thymidin betrug somit 6,392 g (82 %).
R_{f} (SiO₂, Toluol/EtOAc/MeOH 5:4:1) 0,53
UV(MeOH), λₘₐₓ [nm] (log ε): 210 (4,36), 263 (4,05)
¹H-NMR (250 MHz, CDCl₃): 8,11 (s, NH); 7,74 (d, J = 8,2, 1 arom. H v. ClNPEOC); 7,67 (d, J = 8,0, 1 arom. H v. ClNPEOC), 7,39 (t, H-C(4) v. ClNPEOC); 7,38 (s, H-C(6) v. Thymin); 6,36 (t, H-C(1')); 4,44 (m, α-CH₂ v. ClNPEOC, H-C(3'), 2 x H-C(5')); 4,15 (q, H-C(4')); 3,45 (t, β-CH₂ v. ClNPEOC); 2,38 (m, H-C(2')); 2,26 (m, H-C(2')), 2,18 (d, J = 3,9, OH-C(3')); 1,86 (s, CH₃)
Anal. ber. für C₁₉H₂₀ClN₃O₉ (469,83): C 48,57, H 4,29, N 8,94; gef.: C 48,53, H 4,34, N 8,91

### Beispiel 5

### a) 2-(2-Brom-6-nitrophenyl)ethanol [1]

Zu 2-Brom-6-nitrotoluol (1,08 g, 5 mmol) und Paraformaldehyd (150 mg, 5 mmol) in DMSO (2,5 ml, Synthese-Qualität, zusätzlich 2 d über Molekularsieb 4 Å getrocknet) wurde eine Lösung von Kaliumtertiärbutylat (90 mg, 0,8 mmol) in tert.-Butanol (1 ml, Synthese-Qualität, 99 %) gegeben. Nach der Zugabe der Kaliumtertiärbutylat-Lösung trat ein Farbumschlag von gelb nach tief-violett ein. Es wurde 5 min bei Raumtemperatur und 30 min bei 70°C (Ölbadtemperatur) gerührt. Danach ließ man auf Raumtemperatur abkühlen und neutralisierte mit wenigen Tropfen konz. HCl. Das Gemisch wurde mit EtOAc (30 ml) verdünnt und mit H₂O (20 ml) gewaschen. Die wäßrige Phase wurde mit EtOAc (2 x 20 ml) nachextrahiert. Trocknen der organischen Phasen über Na₂SO₄, Filtrieren und Abrotieren des Lösemittels ergaben das Rohprodukt (1,49 g), welches durch Säulenchromatographie (20 g SiO₂, 20 x 2 cm, Lösemittel: PE 45 ml, PE/EtOAc 10:1 110 ml, 8:1 180 ml, 7,5:1 340 ml, 6:1 140 ml) gereinigt wurde. Man erhielt 2-(2-Brom-6-nitrophenyl)ethanol (867 mg, 70 %) als gelben Feststoff.
R_{f} (SiO₂, Toluol/EtOAc 9:1) 0,32
UV(MeOH), λₘₐₓ [nm] (log ε): 204 (4,18), 210 (Schulter, 4,16), 251 (3,48), 293 (Schulter, 3,07)
¹H-NMR (250 MHz, CDCl₃): 7,82 (dd, J = 1,1, 8,0, 1 arom. H); 7,74 (dd, J = 1,1, 8,2, 1 arom. H); 7,26 (m, H-C(4)); 3,96 (t, α-CH₂); 3,30 (t, β-CH₂); 1,75 (s (br), OH)
Anal. ber. für C₈H₈BrNO₃ (246,06): C 39,05, H 3,28, N 5,69; gef.: C 39,09, H 3,26, N 5,56

### Literatur:

[1] Chem. Abstr. 1989, 110, P 75032 k

### b) 2-(2-Brom-6-nitrophenyl)ethoxycarbonylchlorid

Zu einer auf 0°C gekühlten Lösung von Chlorameisensäuretrichlormethylester (442 mg, 2,23 mmol) in THF (5 ml, dest. über CaH₂) wurde in 10 min eine Lösung von 2-(2-Brom-6-nitrophenyl)ethanol (500 mg, 2,03 mmol) und Et₃N (206 mg, 2,03 mmol, dest. über KOH) in THF (5 ml, s.o.) zugegeben. Man ließ 5 min unter Eisbadkühlung und 1 h 45 min bei Raumtemperatur rühren. Das Gemisch wurde über Celite filtriert. Nachwaschen des Filterkuchens mit THF und Abdestillieren des Lösemittels sowie des überschüssigen Reagenses von den vereinigten Filtraten bei 30°C im Hochvakuum ergaben 2-(2-Brom-6-nitrophenyl)-ethoxycarbonylchlorid (625 mg, 99,8 %) als hellbraunes Öl.
R_{f} (SiO₂, PE/EtOAc 19:1) 0,31
UV(MeOH), λₘₐₓ [nm] (log ε): 205 (Schulter, 4,14), 211 (4,16), 254 (3,52), 297 (Schulter, 3,05)
¹H-NMR (250 MHz, CDCl₃): 7,85 (2 arom. H); 7,33 (t, H-C(4)); 4,62 (t, α-CH₂); 3,47 (t, β-CH₂)
Anal. ber. für C₉H₇BrClNO₄ (308,515): C 35,04, H 2,29, N 4,54; gef.: C 35,50, H 2,58, N 4,50

### c) 5'-O-(2-(2-Brom-6-nitrophenyl)ethoxycarbonyl)thymidin

Thymidin (200 mg, 0,83 mmol) wurde mit Pyridin (3 x 3 ml, pro analysi-Qualität, zusätzlich über Molekularsieb 4 Å getrocknet) koevaporiert, in Pyridin (3 ml, s.o.) gelöst und auf -60°C (i-PrOH/N₂) gekühlt. Hierzu tropfte man in 10 min eine Lösung von 2-(2-Brom-6-nitrophenyl)ethoxycarbonylchlorid (354 mg, 1,15 mmol) in CH₂Cl₂ (3 ml, dest. über CaH₂). Man ließ 3 h 40 min unter i-PrOH/N₂ Kühlung (-60 bis -20°C) rühren. Die Reaktionsmischung wurde mit CH₂Cl₂ (10 ml) verdünnt und mit H₂O (10 ml) gewaschen. Die wäßrige Phase wurde mit CH₂Cl₂ (2 x 10 ml) nachextrahiert. Trocknen der organischen Phasen über Na₂SO₄, Filtrieren, Abrotieren des Lösemittels und Koevaporieren mit Toluol (4 x 10 ml) ergaben das Rohprodukt (552 mg). Durch Kristallisation aus wenig CH₂Cl₂ und MeOH konnte 5'-O-(2-(2-Brom-6-nitrophenyl)-ethoxycarbonyl)thymidin (163 mg, 38 %) als farbloser Feststoff isoliert werden. Der Rückstand wurde durch Säulenchromatographie (18 g SiO₂, 11 x 2 cm, Lösemittel: CH₂Cl₂/MeOH 100:1 50 ml, 100:3 103 ml, 100:4 208 ml, 100:5 52 ml) gereinigt. Man eluierte zuerst 3',5'-Bis-O-(2-(2-Brom-6-nitrophenyl)ethoxycarbonyl)thymidin, dann 3'-O-(2-(2-Brom-6-nitrophenyl)ethoxycarbonyl)thymidin und zuletzt 5'-O-(2-(2-Brom-6-nitrophenyl)ethoxycarbonyl)thymidin. Die Produktfraktionen wurden einrotiert und im Hochvakuum getrocknet. Man erhielt 3',5'-Bis-O-(2-(2-Brom-6-nitrophenyl)ethoxycarbonyl)thymidin (62 mg, 10 %) als farblosen Schaum, 3'-O-(2-(2-Brom-6-nitrophenyl)-ethoxycarbonyl)thymidin (8 mg, 2 %) als farbloses Öl und 5'-O-(2-(2-Brom-6-nitrophenyl)ethoxycarbonyl)thymidin (151 mg, 35 %) als farblosen Feststoff. Die Gesamtausbeute an 5'-O-(2-(2-Brom-6-nitrophenyl)ethoxycarbonyl)thymidin betrug somit 314 mg (73 %).
5'-O-(2-(2-Brom-6-nitrophenyl)ethoxycarbonyl)thymidin
R_{f} (SiO₂, Toluol/EtOAc/MeOH 5:4:1) 0,38
UV(MeOH), λₘₐₓ [nm] (log ε): 210 (4,37), 263 (4,06)
¹H-NMR (250 MHz, CDCl₃): 8,35 (s, NH); 7,85 (d, 1 arom. H v. BNPEOC); 7,76 (d, 1 arom. H v. BNPEOC), 7,38 (s, H-C(6) v. Thymin); 7,30 (t, H-C(4) v. BNPEOC, teilweise unter CHCl₃-Signal verborgen); 6,37 (t, H-C(1')); 4,45 (m, H-C(3'), 2 x H-C(5'), α-CH₂ v. BNPEOC)); 4,15 (m, H-C(4')); 3,47 (t, β-CH₂ v. BNPEOC); 2,41 (m, H-C(2'), OH-C(3')); 2,25 (m, H-C(2')); 1,86 (s, CH₃)
Anal. ber. für C₁₉H₂₀BrN₃O₉ (514,285): C 44,37, H 3,92, N 8,17; gef.: C 44,31, H 3,96, N 8,11

### Beispiel 6

### a) 2-(4-Chlor-2-nitrophenyl)ethanol [1, 2, 3]

Ein Gemisch von 4-Chlor-2-nitrotoluol (50 g, 291 mmol) und Paraformaldehyd (3,8 g, 120 mmol) in DMSO (40 ml, Synthese-Qualität, zusätzlich 2 d über Molekularsieb 4 Å getrocknet) wurde mit Triton B (4 ml, 35 % in MeOH) versetzt und 2,5 h bei 90°C gerührt. Man neutralisierte mit wenigen Tropfen konz. HCl, verdünnte mit H₂O (100 ml) und extrahierte mit EtOAc (5 x 150 ml). Die vereinigten organischen Phasen wurden über MgSO₄ getrocknet, filtriert und einrotiert. Der ölige Rückstand wurde durch Sublimation im Hochvakuum (p = 0,1 Torr, Ölbadtemperatur 110°C) gereinigt. Man erhielt 2-(4-Chlor-2-nitrophenyl)ethanol (13,23 g, 55 %) in Form gelber Kristalle.
R_{f} (SiO₂, Toluol/EtOAc 10:1) 0,26
Schmelzpunkt: 61 bis 64°C
UV(MeOH), λₘₐₓ [nm] (log ε): 213 (4,29), 252 (3,61), 294 (Schulter, 3,10)
¹H-NMR (250 MHz, CDCl₃): 7,94 (d, J = 2,2, H-C(3)); 7,53 (dd, H-C(5)); 7,40 (d, H-C(6)); 3,93 (t, α-CH₂); 3,14 (t, β-CH₂); 1,70 (s, OH)
Anal. ber. für C₈H₈ClNO₃ (201,61): C 47,66, H 4,00, N 6,95; gef.: C 47,69, H 4,01, N 6,76

### Literatur:

[1] J. Bakke, Acta Chem. Scand. 1969, 23, 3055
[2] T. Morimoto, I. Hashimoto, H. Yamaoka, Chem. Abstr. 1978, 88, 104880 v
[3] Y. Tsuji, S. Kotachi, K.-T. Huh, Y. Watanabe, J. Org. Chem. 1990, 55, 580

### b) 2-(4-Chlor-2-nitrophenyl)ethoxycarbonylchlorid

In eine Lösung von 2-(4-Chlor-2-nitrophenyl)ethanol (6,8 g, 34 mmol) in THF (50 ml, dest. über CaH₂) wurde bei Raumtemperatur Phosgen eingeleitet. Nach 2,5 h wurde das überschüssige Phosgen sowie das Lösemittel im Hochvakuum abdestilliert. Man erhielt 2-(4-Chlor-2-nitrophenyl)-ethoxycarbonylchlorid (8,53 g, 95 %) als gelbes Öl.
R_{f} (SiO₂, CHCl₃) 0,85
UV(CH₃CN), λₘₐₓ [nm] (log ε): 213 (4,26); 254 (3,63); 300 (Schulter, 3,14)
¹H-NMR (250 MHz, CDCl₃): 8,03 (d, H-C(3)); 7,59 (dd, H-C(5)); 7,36 (d, H-C(6)); 4,62 (t, α-CH₂); 3,32 (β-CH₂)
Anal. ber. für C₉H₇Cl₂NO₄ (264,06): C 40,94, H 2,67, N 5,30; gef.: C 40,97, H 2,69, N 5,00

### c) 5'-O-(2-(4-Chlor-2-nitrophenyl)ethoxycarbonyl)thymidin

Thymidin (150 mg, 0,62 mmol) wurde mit Pyridin (2 x 5 ml, pro analysi-Qualität, zusätzlich über Molekularsieb 4 Å getrocknet) koevaporiert, in Pyridin gelöst und auf -30°C gekühlt. Hierzu tropfte man in 5 min eine Lösung von 2-(4-Chlor-2-nitrophenyl)ethoxycarbonylchlorid (255 mg, 0,97 mmol) in CH₂Cl₂ (3 ml, dest. über CaH₂). Nach 4 h Rühren unter i-PrOH/N₂ Kühlung (-30 bis -15°C) wurde das Gemisch mit CH₂Cl₂ (30 ml) verdünnt und mit H₂O (30 ml) gewaschen. Die wäßrige Phase wurde mit CH₂Cl₂ (2 x 30 ml) nachextrahiert. Die vereinigten organischen Phasen wurden über MgSO₄ getrocknet, filtriert und mit Toluol (4 x 10 ml) und CH₂Cl₂ (20 ml) koevaporiert. Das Rohprodukt wurde durch Säulenchromatographie (SiO₂ 15 x 3 cm, Lösemittel: CH₂Cl₂/MeOH 100:7 700 ml) gereinigt. Man erhielt 5'-O-(4-Chlor-2-nitrophenyl)ethoxycarbonyl)thymidin (219 mg, 75 %) als farblosen Feststoff.
R_{f} (SiO₂, Toluol/EtOAc/MeOH 5:4:1) 0,36
UV(MeOH), λₘₐₓ [nm] (log ε): 212 (4,43), 261 (4,08), 310 (Schulter, 3,07)
¹H-NMR (250 MHz, CDCl₃): 8,75 (s (br), NH); 7,94 (d, H-C(3) v. CNPEOC); 7,52 (dd, H-C(5)); 7,31 (m, H-C(6) v. CNPEOC, H-C(6) von Thymin); 6,32 (t, H-C(1')); 4,41 (m, H-C(3'), 2 x H-C(5'), α-CH₂ v. CNPEOC); 4,12 (q, H-C(4')); 3,27 (t, β-CH₂ v. CNPEOC); 2,75 (s, (br), OH-C(3')); 2,39 (m, H-C(2')), 2,19 (m, H-C(2')); 1,86 (S, CH₃)
Anal. ber. für C₁₉H₂₀ClN₃O₉ (469,83): C 48,57, H 4,29, N 8,94; gef.: C 48,87, H 4,56, N 8,64

### Beispiel 7

### a) 2-(5-Methoxy-2-nitrophenyl)ethanol [1, 2]

Ein Gemisch aus 5-Methoxy-2-nitrotoluol (25 g, 150 mmol) und Paraformaldehyd (2,3 g, 73 mmol) in DMSO (20 ml, Synthese-Qualität, zusätzlich 2 d über Molekularsieb 4 Å getrocknet) wurde bei 80°C mit Triton B (2 ml, 35 % in MeOH) versetzt. Nach 2,5 h Rühren bei dieser Temperatur ließ man auf Raumtemperatur abkühlen und neutralisierte mit wenigen Tropfen konz. HCl. Das Gemisch wurde mit H₂O (50 ml) verdünnt und mit EtOAc (5 x 100 ml) extrahiert. Die organischen Phasen wurden über MgSO₄ getrocknet, filtriert und einrotiert. Der Rückstand wurde einer Destillation im Hochvakuum (p = 0,1 Torr) unterworfen, wobei bei 70°C eine Fraktion mit Edukt (12,1 g) erhalten wurde. Das restliche Rohprodukt wurde durch Säulenchromatographie (240 g SiO₂, Lösemittel: Toluol/EtOAc 8:1 1400 ml, 7:1 1600 ml, 6:1 400 ml, 5:1 400 ml, 3:1 400 ml, 2:1 600 ml und 1:1 600 ml) gereinigt. Man erhielt 2-(5-Methoxy-2-nitrophenyl)ethanol (6,87 g, 48 %) als gelbes Öl.
R_{f} (SiO₂, Toluol/EtOAc 1:1) 0,43
UV(MeOH), λₘₐₓ [nm] (log ε): 204 (4,00), 231 (3,82); 301 (3,84)
¹H-NMR (250 MHz, CDCl₃) : 8,04 (dd, H-C(4); 6,82 (m, H-C(3), H-C(6)); 3,94 (q, α-CH₂); 3,87 (s, OCH₃); 3,20 (t, β-CH₂); 1,64 (s (br), OH)
Anal. ber. für C₉H₁₁NO₄ (197,19): C 54,82, H 5,62, N 7,10; gef.: C 54,78, H 5,86, N 7,00

### Literatur:

[1] T. Morimoto, I. Hashimoto, H. Yamaoka, Chem. Abstr. 1978, 88, 104880 v
[2] Y. Tsuji, S. Kotachi, K.-T. Huh, Y. Watanabe, J. Org. Chem. 1990, 55, 580

### b) 2-(5-Methoxy-2-nitrophenyl)ethoxycarbonylchlorid

In eine Lösung von 2-(5-Methoxy-2-nitrophenyl)ethanol (3,0 g, 15 mmol) in THF (40 ml, dest. über CaH2) wurde bei Raumtemperatur unter Rühren Phosgen eingeleitet. Nach 2,5 h wurden das überschüssige Phosgen sowie das Lösemittel im Hochvakuum abdestilliert. Man erhielt 2-(5-Methoxy-2-nitrophenyl)ethoxycarbonylchlorid (3,72 g, 96 %) als gelbes Öl.
R_{f} (SiO₂, CHCl₃) 0,79
UV(CH₃CN), λₘₐₓ [nm] (log ε) : 222 (Schulter, 3,87), 230 (3,90), 303 (3,87)
¹H-NMR (250 MHz, CDCl₃): 8,12 (d, H-C(3)); 6,88 (dd, H-C(4)); 6,79 (d, J = 2,8, H-C(6)), 4,63 (t, α-CH₂); 3,89 (s, OCH₃); 3,35 (t, β-CH₂)
Anal. ber. für C₁₀H₁₀ClNO₅: C 46,26, H 3,88, N 5,39; gef.: C 46,42, H 4,00, N 5,50

### c) 5'-O-(2-(5-Methoxy-2-nitrophenyl)ethoxycarbonyl)thymidin

Thymidin (150 mg, 0,62 mmol) wurde mit Pyridin (2 x 5 ml, pro analysi-Qualität, zusätzlich über Molekularsieb 4 Å getrocknet) koevaporiert, in Pyridin (5 ml, s.o.) gelöst und auf -30°C (i-PrOH/N₂) gekühlt. Hierzu tropfte man in 5 min eine Lösung von 2-(5-Methoxy-2-nitrophenyl)-ethoxycarbonylchlorid (250 mg, 0,96 mmol) in CH₂Cl₂ (5 ml, dest. über CaH₂). Nach insgesamt 4 h Rühren unter i-PrOH/N₂ Kühlung (-30 bis -15°C) wurde das Gemisch mit CH₂Cl₂ (30 ml) verdünnt und mit H₂O (30 ml) gewaschen. Die wäßrige Phase wurde mit CH₂Cl₂ (2 x 30 ml) nachextrahiert. Trocknen der organischen Phasen über Na₂SO₄, Filtrieren, Abrotieren des Lösemittels und Koevaporieren mit Toluol (4 x 10 ml) und CH₂Cl₂ (10 ml) ergaben das Rohprodukt, welches durch Säulenchromatographie (SiO₂, 16 x 3 cm, CH₂Cl₂/MeOH 100:6 800 ml) gereinigt wurde. Man erhielt 5'-O-(2-(5-Methoxy-2-nitrophenyl)ethoxycarbonyl)thymidin (205 mg, 71 %) als farblosen Feststoff.
R_{f} (SiO₂, Toluol/EtOAc/MeOH 5:4:1) 0,32
UV(MeOH), λₘₐₓ [nm] (log ε): 205 (4,30), 234 (Schulter, 3,94), 270 (4,09), 302 (Schulter, 3,84)
¹H-NMR (250 MHz, CDCl₃) : 8,79 (s, NH); 8,06 (H-C(3) v. MNPEOC); 7,34 (s, H-C(6) v. Thymin); 6,85 (dd, H-C(4) v. MNPEOC); 6,77 (d, J = 2,7, H-C(6) v. MNPEOC); 6,32 (t, H-C(1')); 4,43 (m, H-C(3'), 2 x H-C(5'), α-CH₂ v. MNPEOC); 4,12 (q, H-C(4')); 3,85 (s, OCH₃); 3,33 (m, β-CH₂ v. MNPEOC); 2,75 (d, OH-C(3')); 2,38 (m, H-C(2')); 2,18 (m, H-C(2')); 1,83 (s, CH₃)
Anal. ber. für C₂₀H₂₃N₃O₁₀ (465, 42): C 51,61, H 4,98, N 9,03; gef.: C 51,31, H 5,09, N 8,63

### Beispiel 8

### a) 2-(2,4-Dichlor-6-nitrophenyl)ethanol

Zu einem Gemisch von 2,4-Dichlor-6-nitrotoluol (1,03 g, 5 mmol) und Paraformaldehyd (150 mg, 5 mmol) in DMSO (2,5 ml, Synthese-Qualität, zusätzlich 2 d über Molekularsieb 4 Å getrocknet) wurde eine Lösung von Kaliumtertiärbutylat (90 mg, 0,8 mmol) in tert.-Butanol (1 ml, Synthese-Qualität, 99 %) gegeben. Nach der Zugabe der Kaliumtertiärbutylat-Lösung trat ein Farbumschlag von gelb nach tief-violett ein: Es wurde 5 min bei Raumtemperatur und 30 min bei 70 bis 80°C (Ölbadtemperatur) gerührt. Danach ließ man auf Raumtemperatur abkühlen und neutralisierte mit wenigen Tropfen konz. HCl. Das Gemisch wurde mit EtOAc (30 ml) verdünnt und mit H₂O (30 ml) gewaschen. Die wäßrige Phase wurde mit EtOAc (2 x 30 ml) nachextrahiert. Trocknen der organischen Phasen über Na₂SO₄, Filtrieren und Abrotieren des Lösemittels ergaben das Rohprodukt, welches durch Säulenchromatographie (20 g SiO₂, 12 x 2 cm, Lösemittel: PE 30 ml, PE/EtOAc 10:1 110 ml, 9:1 100 ml, 8:1 360 ml, 7:1 80 ml) gereinigt wurde. Man erhielt 2-(2,4-Dichlor-6-nitrophenyl)ethanol (769 mg, 65 %) als gelben Feststoff.
R_{f} (SiO₂, Toluol/EtOAc 9:1) 0,41
UV(MeOH), λₘₐₓ [nm] (log ε) : 205 (4,32), 218 (4,20), 254 (Schulter, 3,40), 292 (3,08)
¹H-NMR (250 MHz, CDCl₃): 7,73 (d, J = 2,0, 1 arom H); 7,65 (d, J = 2,0, 1 arom. H); 3,92 (m, α-CH₂); 3,26 (t, β-CH₂); 1,70 (s (br), OH)
Anal. ber. für C₈H₇Cl₂NO₃ (236,054): C 40,71, H 2,99, N 5,93; gef.: C 40,36, H 2,96, N 5,85

### b) 2-(2,4-Dichlor-6-nitrophenyl)ethoxycarbonylchlorid

Zu einer auf 0°C gekühlten Lösung von Chlorameisensäuretrichlormethylester (503 mg, 2,5 mmol) in THF (5 ml, dest. über CaH2) wurde in 5 min eine Lösung von 2-(2,4-Dichlor-6-nitrophenyl)ethanol (500 mg, 2,12 mmol) und Et₃N (214 mg, 2,12 mmol, dest. über KOH) in THF (5 ml, s.o.) zugegeben. Man ließ 1 h unter Eisbadkühlung und 2 h bei Raumtemperatur rühren. Das Gemisch wurde dann über Celite filtriert. Nachwaschen des Filterkuchens mit THF und Abdestillieren des Lösemittels und des überschüssigen Reagenses von den vereinigten Filtraten bei 30°C im Hochvakuum ergaben 2-(2,4-Dichlor-6-nitrophenyl)-ethoxycarbonylchlorid (597 mg, 94 %) als hellgelben Feststoff.
R_{f} (SiO₂, PE/EtOAc 19:1) 0,57
UV(MeOH), λₘₐₓ [nm] (log ε): 204 (4,35), 217 (4,25), 252 (Schulter, 3,47), 295 (3,10)
¹H-NMR (250 MHz, CDCl₃): 7,82 (d, J = 1,8, 1 arom. H); 7,70 (d, J = 1,8, 1 arom. H); 4,59 (t, α-CH₂); 3,42 (t, β-CH₂)
Anal. ber. für C₉H₆Cl₃NO₄ (298,509): C 36,21, H 2,03, N 4,69; gef.: C 36,37, H 2,30, N 4,60

### c) 5'-O-(2-(2,4-Dichlor-6-nitrophenyl)ethoxycarbonyl)thymidin

Thymidin (200 mg, 0,83 mmol) wurde mit Pyridin (3 x 3 ml, pro analysi-Qualität, zusätzlich über Molekularsieb 4 Å getrocknet) koevaporiert, in Pyridin (3 ml, s.o.) gelöst und auf -60°C (i-PrOH/N₂) gekühlt. Hierzu tropfte man in 15 min eine Lösung von 2-(2,4-Dichlor-6-nitrophenyl)-ethoxycarbonylchlorid (320 mg, 1,07 mmol) in CH₂Cl₂ (3 ml, dest. über CaH₂). Nach insgesamt 6 h Rühren unter i-PrOH/N₂ Kühlung (-60 bis -15°C) wurde das Gemisch mit CH₂Cl₂ (10 ml) verdünnt und mit H₂O (10 ml) gewaschen. Die wäßrige Phase wurde mit CH₂Cl₂ (2 x 10 ml) nachextrahiert. Trocknen der organischen Phasen über Na₂SO₄, Filtrieren, Abrotieren des Lösemittels und Koevaporieren mit Toluol (4 x 10 ml) ergaben das Rohprodukt (543 mg), welches durch Säulenchromatographie (20 g SiO₂, 12,5 x 2,1 cm, Lösemittel: CH₂Cl₂/MeOH 100:1 50 ml, 100:2 204 ml, 100:3 360 ml) gereinigt wurde. Man eluierte zuerst 3',5'-Bis-O-(2-(2,4-Dichlor-6-nitrophenyl)ethoxycarbonyl)thymidin, dann 3'-O-(2-(2,4-Dichlor-6-nitrophenyl)ethoxycarbonyl)thymidin und 5'-0-(2-(2,4-Dichlor-6-nitrophenyl)ethoxycarbonyl)thymidin. Die Produktfraktionen wurden einrotiert und im Hochvakuum getrocknet. Man erhielt 3',5'-Bis-O-(2-(2,4-Dichlor-6-nitrophenyl)ethoxycarbonyl)thymidin (53 mg, 8 %) als hellgelben Schaum sowie 3'-O-(2-(2,4-Dichlor-6-nitrophenyl)ethoxycarbonyl)thymidin (14 mg, 3 %) und 5'-O-(2-(2,4-Dichlor-6-nitrophenyl)ethoxycarbonyl)thymidin (339 mg, 81 %) jeweils als farblosen Schaum. 5'-O-(2-(2,4-Dichlor-6-nitrophenyl)ethoxycarbonyl)thymidin:
R_{f} (SiO₂, Toluol/EtOAc/MeOH 5:4:1) 0,40
UV(MeOH), λₘₐₓ [nm] (log ε): 203 (4,51), 214 (Schulter, 4,38), 263 (4,02), 304 (Schulter, 3,06)
¹H-NMR (250 MHz, CDCl₃): 8,91 (s, NH); 7,75 (d, J = 2,1, 1 arom. H v. DCNPEOC); 7,68 (d, J = 2,1, 1 arom. H v. DCNPEOC); 7,37 (s, H-C(6) v. Thymin); 6,37 (t, H-C(1')); 4,63 (m, H-C(3'), 2 x H-C(5'), α-CH₂ v. DClNPEOC); 4,16 (m, H-C(4')); 3,40 (t, β-CH₂ v. DClNPEOC); 2,86 (d, OH-C(3')); 2,42 (m, H-C(2')); 2,24 (m, H-C(2')); 1,89 (s, CH₃)
Anal. ber. für C₁₉H₁₉Cl₂N₃O₉ (504,279): C 45,25, H 3,80, N 8,33, gef.: C 45,02, H 3,89, N 8,04

### Beispiel 9

### a) 2-(4,5-Dimethoxy-2-nitrophenyl)ethanol [1]

Zu einer auf -10°C (Viehsalz/Eis) gekühlten Mischung aus Homoveratrylalkohol (3,02 g, 16,6 mmol) in Eisessig (30 ml) wurde in 6 min unter Rühren konz. HNO₃ (4,8 ml, 65 %, d = 1,4) zugetropft. Anschließend ließ man in 30 min auf 23°C erwärmen. Nach 1 h Rühren bei dieser Temperatur wurde das Gemisch mit H₂O (30 ml) verdünnt, mit NaHCO₃ neutralisiert und mit EtOAc (3 x 30 ml) extrahiert. Die vereinigten organischen Phasen wurden über Na₂SO₄ getrocknet, filtriert und einrotiert. Der Rückstand (3,25 g) wurde durch Säulenchromatographie (90 g SiO₂ Toluol/EtOAc 4:1 500 ml, Toluol/EtOAc 3:1 400 ml, Toluol/EtOAc 2:1 300 ml) gereinigt. Man erhielt 2-(4,5-Dimethoxy-2-nitrophenyl)ethanol (2,13 g, 56 %) als gelben Feststoff.
Rf (SiO₂, Toluol/EtOAc 2:1) 0,24
UV(MeOH), λₘₐₓ [nm] (log ε): 202 (4,18), 216 (4,06), 242 (3,97), 297 (3,63), 340 (3,70)
¹H-NMR-Spektrum (250 MHz, CDCl₃): 7,61 (s, H-C(3)); 6,80 (s, H-C(6)); 3,97 (s, OCH₃); 3,96 (m, α-CH₂, versteckt); 3,95 (s, OCH₃); 3,21 (t, β-CH₂), 1,70 (s (br), OH)
Anal. ber. für C₁₀H₁₃NO₅ (227.216): C 52,86, H 5,77, N 6,16; gef.: C 52,87, H 5,82, N 6,12

### Literatur

[1] E. McDonald, R. D. Wylie, Tetrahedron 1979, 35, 1415

### b) 2-(4,5-Dimethoxy-2-nitrophenyl)ethoxycarbonylchlorid

Chlorameisensäuretrichlormethylester (0,26 ml, 2,2 mmol) in THF (5 ml, dest. über CaH₂) wurde mittels eines Eisbades auf 0°C gekühlt. Hierzu tropfte man in 10 min eine Lösung von 2-(4,5-Dimethoxy-2-nitrophenyl)ethanol (500 mg, 2,2 mmol) und Et₃N (218 mg, 0,3 ml, 2,2 mmol, dest. über CaH₂) in THF (15 ml, dest. über CaH₂). Anschließend entfernte man das Eisbad und ließ bei Raumtemperatur weiterrühren. Nach 30 min Rühren versetzte man die Reaktionsmischung mit einer Spatelspitze Aktivkohle. Das Reaktionsgemisch wurde nach weiteren 2,5 h Rühren bei Raumtemperatur über Celite abgesaugt. Man destillierte das Lösemittel sowie überschüssiges Reagens im Hochvakuum vom Filtrat ab und erhielt 2-(4,5-Dimethoxy-2-nitrophenyl)ethoxycarbonylchlorid (624 mg, 98 %) als gelbbraunen Feststoff.
R_{f} (SiO₂, Toluol/EtOAc 2:1) 0,77
UV(MeOH), λₘₐₓ [nm] (log ε) : 203 (4,18), 217 (4,08), 242 (4,01), 297 (3,67), 339 (3,73)
¹H-NMR (250 MHz, CDCl₃) : 7,67 (s, H-C(3)); 6,74 (s, H-C(6)); 4,66 (t, α-CH₂); 3,99 (s, OCH₃); 3,96 (s, OCH₃); 3,36 (t, β-CH₂)
Anal. ber. für C₁₁H₁₂NO₆Cl (289,671): C 45,61, H 4,18, N 4,84; gef.: C 45,59, H 4,26, N 4,94

### c) 5'-O-(2-(4,5-Dimethoxy-2-nitrophenyl)ethoxycarbonyl)-thymidin

Thymidin (200 mg, 0,83 mmol) wurde mit Pyridin (2 x 2 ml, pro analysi-Qualität, zusätzlich über Molekularsieb 4 Å getrocknet) koevaporiert, in Pyridin (2,4 ml, s.o.) gelöst und auf -60°C (i-PrOH/N₂) gekühlt. Hierzu tropfte man in 15 min eine Lösung von 2-(4,5-Dimethoxy-2-nitrophenyl)-ethoxycarbonylchlorid (361 mg, 1,25 mmol) in CH₂Cl₂ (2,4 ml, dest. über CaH₂). Nach insgesamt 6,5 h Rühren unter i-PrOH/N₂ Kühlung (-40 bis -15°C) wurde das Gemisch mit CH₂Cl₂ (10 ml) verdünnt und mit H₂O (10 ml) gewaschen. Die wäßrige Phase wurde mit CH₂Cl₂ (2 x 10 ml) nachextrahiert. Trocknen der organischen Phasen über Na₂SO₄, Filtrieren, Abrotieren des Lösemittels und Koevaporieren mit Toluol (4 x 20 ml) ergaben das Rohprodukt (530 mg), welches durch Säulenchromatographie (20 g SiO₂, CH₂Cl₂/MeOH 100:3 309 ml, 100:4 104 ml, 100:5 160 ml) gereinigt wurde. Man eluierte zuerst 3',5'-Bis-O-(2-(4,5-Dimethoxy-2-nitrophenyl) ethoxycarbonyl) thymidin und dann 5'-O-(2-(4,5-Dimethoxy-2-nitrophenyl)ethoxycarbonyl)thymidin. Die Produktfraktionen wurden einrotiert und im Hochvakuum getrocknet. Man erhielt 3',5'-Bis-O-(2-(4,5-Dimethoxy-2-nitrophenyl)ethoxycarbonyl)thymidin (154 mg, 25 %) und 5'-O-(2- (4,5-Dimethoxy-2-nitrophenyl) ethoxycarbonyl) thymidin (272 mg, 66 %) jeweils als hellgelbe Feststoffe.
5'-O-(2-(4,5-Dimethoxy-2-nitrophenyl)ethoxycarbonyl)-thymidin:
R_{f} (SiO₂, Toluol/EtOAc/MeOH 5:4:1) 0,23
UV(MeOH), λₘₐₓ [nm] (log ε): 203 (4,33), 212 (Schulter, 4,30), 247 (4,13), 267 (4,03), 343 (3,69)
¹H-NMR (250 MHz, CDCl₃): 9,10 (s, NH); 7,62 (s, H-C(3)); 7,37 (d, J = 1,2, H-C(6) v. Thymin); 6,73 (s, H-C(6)); 6,35 (t, H-C(1')); 4,44 (m, H-C(3'), 2 x H-C(5'), α-CH₂ v. DMNPEOC); 4,15 (m, H-C(4')); 3,96 (s, OCH₃) ; 3,94 (s, OCH₃); 3,33 (dt, β-CH₂ v. DMNPEOC); 3,06 (s (br), OH-C(3')); 2,42 (m, H-C(2')); 2,20 (m, H-C(2')); 1,88 (d, J = 0,9, CH₃)
Anal. ber. für C₂₁H₂₅N₃O₁₁ (495,441): C 50,91, H 5,09, N 8,48, gef.: C 50,94, H 5,09, N 8,32

### Beispiel 10

### a) 2-(2-Nitrophenyl)propanol [1, 2]

Zu 2-Nitroethylbenzol (3,02 g, 20 mmol) und Paraformaldehyd (600 mg, 20 mmol) in DMSO (10 ml, Synthese-Qualität, zusätzlich 2 d über Molekularsieb 4 Å getrocknet) wurde eine Lösung von Kaliumtertiärbutylat (360 mg, 3,2 mmol) in tert.-Butanol (4 ml, dest. über CaH₂) gegeben. Es wurde 15 min bei Raumtemperatur und 1 h 45 min bei 70°C (Ölbadtemperatur) gerührt. Danach ließ man auf Raumtemperatur abkühlen und neutralisierte mit wenigen Tropfen konz. HCl. Das Gemisch wurde mit EtOAc (100 ml) verdünnt und mit gesättigter NaCl-Lsg. (60 ml) gewaschen. Die wäßrige Phase wurde mit EtOAc (2 x 100 ml) nachextrahiert. Trocknen der organischen Phasen über Na₂SO₄, Filtrieren und Abrotieren des Lösemittels ergaben das Rohprodukt (5,06 g), welches durch Säulenchromatographie (80 g SiO₂, 19 x 3,4 cm, Lösemittel: Toluol 150 ml, Toluol/EtOAc 8:1 270 ml, 7:1 240 ml, 6:1 280 ml, 5:1 180 ml) gereinigt wurde. Man erhielt 2-(2-Nitrophenyl)propanol (2,539 g, 70 %) als hellgelbes Öl.
R_{f} (SiO₂, Toluol/EtOAc 9:1) 0,25
UV(MeOH), λₘₐₓ [nm] (log ε): 206 (4,08), 220 (Schulter, 3,75), 254 (3,53), 285 (Schulter, 3,27)
¹H-NMR (250 MHz, CDCl₃): 7,75 (dd, J = 1,2, 8,1, 1 arom. H, ortho zu NO₂); 7,55 (m, 2 arom. H); 7,36 (m, 1 arom. H); 3,80 (m, α-CH₂); 3,52 (sextett, β-CH); 1,67 (s (br), OH); 1,33 (d, J = 6,9, CH₃)
Anal. ber. für C₉H₁₁NO₃ (181,191): C 59,66, H 6,12, N 7,73; gef.: C 59,55, H 6,12, N 7,90

### Literatur:

[1] J. Org. Chem. 1986, 3143
[2] Chem. Abstr. 1989, 110, P 75032 k.

### b) 2-(2-Nitrophenyl)propoxycarbonylchlorid

Zu einer auf 0°C gekühlten Lösung von Chlorameisensäuretrichlormethylester (655 mg, 3,3 mmol) in THF (6,75 ml, dest. über CaH₂) wurde in 5 min eine Lösung von 2-(2-Nitrophenyl)propanol (500 mg, 2,76 mmol) und Et₃N (279 mg, 0,385 ml, 2,76 mmol, dest. über KOH) in THF (6,75 ml, s.o.) zugegeben. Man ließ 1 h unter Eisbadkühlung und 1 h bei Raumtemperatur rühren. Das Gemisch wurde über Celite filtriert. Nachwaschen des Filterkuchens mit THF und Abdestillieren des Lösemittels sowie des überschüssigen Reagenses von den vereinigten Filtraten bei 30°C im Hochvakuum ergaben 2-(2-Nitrophenyl)propoxycarbonylchlorid (644 mg, 96 %) als hellbraunes Öl.
R_{f} (SiO₂, PE/EtOAc 19:1) 0,24
UV(MeOH), λₘₐₓ [nm] (log ε): 205 (4,07); 218 (Schulter, 3,75), 251 (3,59)
¹H-NMR (250 MHz, CDCl₃): 7,84 (dd, J = 1,2, 8,0, 1 arom. H, ortho zu NO₂); 7,62 (m, 1 arom. H); 7,44 (m, 2 arom. H); 4,50 (d, J = 6,3, α-CH₂); 3,80 (sextett β-CH₂); 1,42 (d, J = 7,0, CH₃)
Anal. ber. für C₁₀H₁₀ClNO₄ (243,646): C 49,30, H 4,14, N 5,75; gef.: C 49,71, H 4,32, N 5,70

### c) 5'-O-(2-(2-Nitrophenyl)propoxycarbonyl)thymidin

Thymidin (1 g, 4,1 mmol) wurde mit Pyridin (3 x 15 ml, pro analysi-Qualität, zusätzlich über Molekularsieb 4 Å getrocknet) koevaporiert, in Pyridin (15 ml, s.o.) gelöst und auf -60°C (i-PrOH/N₂) gekühlt. Hierzu tropfte man in 30 min eine Lösung von 2-(2-Nitrophenyl)propoxycarbonylchlorid (1,31 g, 5,38 mmol) in CH₂Cl₂ (18 ml, dest. über CaH₂). Man ließ weitere 6 h unter i-PrOH/N₂ Kühlung (-60 bis -20°C) rühren. Die Reaktionsmischung wurde mit CH₂Cl₂ (50 ml) verdünnt und mit H₂O (50 ml) gewaschen. Die wäßrige Phase wurde mit CH₂Cl₂ (3 x 50 ml) nachextrahiert. Trocknen der organischen Phasen über Na₂SO₄, Filtrieren, Abrotieren des Lösemittels und Koevaporieren mit Toluol (4 x 15 ml) ergaben das Rohprodukt (2,22 g), welches durch Säulenchromatographie (80 g SiO₂, 19 x 3,4 cm, Lösemittel: CH₂Cl₂/MeOH 100:1 101 ml, 100:2 204 ml, 100:3 206 ml, 100:3,5 207 ml, 100:4 520 ml, 100:5 53 ml, 100:6 53 ml) gereinigt wurde. Man eluierte zuerst 3',5'-Bis-O-(2-(2-Nitrophenyl)propoxycarbonyl)thymidin, dann 3'-O-(2-(2-Nitrophenyl)propoxycarbonyl)thymidin und zuletzt 5'-O-(2-(2-Nitrophenyl)propoxycarbonyl)thymidin. Nach Einrotieren der Produktfraktionen und Trocknen im Hochvakuum erhielt man 3',5'-Bis-O-(2-(2-Nitrophenyl)propoxycarbonyl)thymidin (145 mg, 5 %) als hellgelben Schaum sowie 3'-O-(2-(2-Nitrophenyl)propoxycarbonyl)thymidin (71 mg, 4 %) und 5'-O-(2-(2-Nitrophenyl)propoxycarbonyl)thymidin (1,307 g, 71 %) jeweils als farblosen Schaum.
5'-O-(2-(2-Nitrophenyl)propoxycarbonyl)thymidin:
R_{f} (SiO₂, Toluol/EtOAc/MeOH 5:4:1) 0,43
UV(MeOH), λₘₐₓ [nm] (log ε): 207 (4,30), 263 (4,07)
¹H-NMR (250 MHz, CDCl₃): 8,66 (s, NH, Diastereomer); 8,64 (s, NH, Diastereomer); 7,77 (m, 1 arom. H v. NPPOC); 7,59 (t, 1 arom. H v. NPPOC); 7,43 (m, 2 arom. H v. NPPOC); 7,33 (s, H-C(6) v. Thymin, Diastereomer); 7,30 (s, H-C(6) v. Thymin, Diastereomer), 6,34 (t, H-C(1'), Diastereomer); 6,32 (t, H-C(1'), Diastereomer); 4,29 (m, H-C(3'), H-C(4'), 2 x H-C(5'), α-CH₂ v. NPPOC); 3,80 (m, β-CH v. NPPOC); 2,62 (d, J = 4,2, OH-C(3'), Diastereomer); 2,60 (d, J = 4,4, OH-C(3'), Diastereomer); 2,39 (m, H-C(2')); 2,18 (m, H-C(2')); 1,86 (s, CH₃ v. Thymin, Diastereomer); 1,75 (s, CH₃ v. Thymin, Diastereomer); 1,38 (d, J = 7,0, CH₃ v. NPPOC, Diastereomer); 1,37 (d, J = 7,0, CH₃ v. NPPOC, Diastereomer)
Anal. ber. für C₂₀H₂₃N₃O₉ (449,416): C 53,45, H 5,16, N 9,35; gef.: C 53,14, H 5,21, N 9,16

### Beispiel 11

### a) 2-Chlor-2-(2-nitrophenyl)ethanol

Eine Lösung von 2-Nitrobenzylchlorid (4,3 g, 25 mmol) in DMSO (3,5 ml, Synthese-Qualität, zusätzlich 2 d über Molekularsieb 4 Å getrocknet) wurde mit Paraformaldehyd (750 mg, 25 mmol) und DBU (0,5 ml, 3,3 mmol) versetzt und bei Raumtemperatur 20 min gerührt. Der pH-Wert der Reaktionsmischung wurde mit wenigen Tropfen konz. AcOH auf etwa pH 3 eingestellt. Das Gemisch wurde mit CH₂Cl₂ (120 ml) verdünnt, mit H₂O gewaschen (80 ml) und mit CH₂Cl₂ (2 x 100 ml) nachextrahiert. Die organischen Phasen wurden über Na₂SO₄ getrocknet, filtriert und einrotiert. Das Rohprodukt (5,543 g) wurde durch Säulenchromatographie (150 g SiO₂, 22 x 4,1 cm, Lösemittel: Toluol 150 ml, Toluol/EtOAc 25:1 260 ml, 15:1 320 ml, 10:1 330 ml, 5:1 720 ml, 4:1 250 ml) gereinigt. Man eluierte zuerst das Edukt (2,587 g, 60 %), dann eine Mischfraktion (267 mg) und zuletzt 2-Chlor-2-(2-nitrophenyl)ethanol (1,178 g, 23 % bzw. 61 % bezüglich des umgesetzten Edukts). Die erhaltene Mischfraktion (267 mg) wurde durch eine weitere Säulenchromatographie (8 g SiO₂, 15 x 1,2 cm; Lösemittel: Toluol 50 ml, Toluol/EtOAc 100:1 50 ml) gereinigt. Es wurde zuerst weiteres Edukt (44 mg, 1 %) und dann 2-Nitrostyrolepoxid (68 mg, 2 % bzw. 4 % bzgl. des umgesetzten Edukts) eluiert. Die Gesamtmenge an zurückisoliertem Edukt betrug 2,631 g (61 %).
2-Chlor-2-(2-nitrophenyl)ethanol (gelber Feststoff):
R_{f} (SiO₂, CH₂Cl₂) 0,23
Schmelzpunkt: 49 bis 50°C
UV(MeOH), λₘₐₓ [nm] (log ε): 209 (4,11), 254 (3,64), 332 (Schulter, 2,70)
¹H-NMR (250 MHz, CDCl₃) : 7,94 (dd, J = 1,3, 8,3, 1 arom. H); 7,89 (dd, J = 1,3, 7,9, 1 arom. H); 7,69 (m, 1 arom. H); 7,51 (m, 1 arom. H); 5,73 (dd, J = 4,6, 6,8, β-CH) ; 4,11 (dd, J = 4,6, 12,1, α-CH) ; 4,00 (dd, J = 6,8, 12,0, α-CH); 2,14 (s, OH)
¹H-NMR (250 MHz, DMSO-d₆): 7,94 (dd, J = 1,2, 8,1, 1 arom. H); 7,86 (dd, J = 1,5, 7,9, 1 arom. H); 7,77 (dt, J = 1,2, 7,4, 1 arom. H); 7,60 (dt, J = 1,6, 8,3, 1 arom. H); 5,43 (t, J = 6,4, β-CH; s (br), teilweise versteckt, OH), 3,86 (d, J = 6,4, α-CH₂)
Anal. ber. für C₈H₈ClNO₃ (201,609): C 47,66, H 4,00, N 6,95; gef.: C 48,01, H 4,11, N 7,00

### b) 2-Chlor-2-(2-nitrophenyl)ethoxycarbonylchlorid

Eine Lösung von Diphosgen (1,7 ml, 14 mmol) in THF (10 ml, dest. über CaH2) wurde mittels eines Eisbades auf etwa 0°C gekühlt. Hierzu wurde eine Lösung von 2-Chlor-2-(2-nitrophenyl)ethanol (705 mg, 3,5 mmol) und Et₃N (354 mg, 0,485 ml, 3,5 mmol) in THF (10 ml, dest. über CaH₂) in ca. 30 min gegeben. Nach einer weiteren Stunde Rühren wurde das Eisbad entfernt. Man ließ noch 3 h bei Raumtemperatur rühren. Das Gemisch wurde über Celite abfiltriert. Der Niederschlag wurde mit THF (10 ml, dest. über CaH₂) nachgewaschen. Die vereinigten Filtrate wurden einrotiert und anschließend im Hochvakuum bei Raumtemperatur getrocknet. Man erhielt 2-Chlor-2-(2-nitrophenyl)ethoxycarbonylchlorid (896 mg, 97 %) als hellbraunes Öl.
Rf (SiO₂, CH₂Cl₂) 0,82
UV(MeOH), λₘₐₓ [nm] (log ε): 208 (4,11); 253 (3,66)
¹H-NMR (250 MHz, CDCl₃): 8,01 (dd, J = 1,1, 8,1, 1 arom. H); 7,93 (dd, J = 1,1, 7,9, 1 arom. H); 7,74 (dt, J = 1,1, 8,2, 1 arom. H); 7,57 (m, 1 arom. H); 5,89 (dd, J = 4,9, 6,4, β-CH); 4,80 (dd, J = 6,5, 11,4, α-CH); 4,73 (dd, J = 4,9, 11,5, α-CH)
Anal. ber. für C₉H₇Cl₂NO₄ (264,064): C 40,94, H 2,67, N 5,30; gef.: C 41,39, H 2,89, N 5,38

### c) 5'-O-(2-Chlor-2-(2-nitrophenyl)ethoxycarbonyl)thymidin

Thymidin (250 mg, 1,03 mmol) wurde mit Pyridin (2 x 3 ml, pro analysi-Qualität, zusätzlich über Molekularsieb 4 Å getrocknet) koevaporiert, in Pyridin (3,5 ml, s.o.) gelöst und auf ca. -40°C (i-PrOH/N₂) gekühlt. Hierzu wurde in ca. 30 min eine Lösung von 2-Chlor-2-(2-nitrophenyl)-ethoxycarbonylchlorid (356 mg, 1,35 mmol) in CH₂Cl₂ (3,5 ml, dest. über CaH₂) gegeben. Man ließ weitere 4 h 45 min unter i-PrOH/N₂ Kühlung (-40 bis -10°C) rühren. Die Reaktionsmischung wurde mit H₂O (10 ml) verdünnt und mit CH₂Cl₂ (4 x 10 ml) extrahiert. Die organischen Phasen wurden über Na₂SO₄ getrocknet, abfiltriert, einrotiert und mit Toluol (4 x 10 ml) koevaporiert. Das Rohprodukt (665 mg) wurde durch Säulenchromatographie (20 g SiO₂, 12 x 2,1 cm, Lösemittel: CH₂Cl₂ 100 ml, CH₂Cl₂/MeOH 100:1 101 ml, 100:2 102 ml, 100:2,5 102 ml, 100:3 206 ml, 100:4 104 ml, 100:5 105 ml) gereinigt. Man eluierte zuerst Bis-(2-Chlor-2-(2-nitro-phenyl)ethyl)carbonat (60 mg, 10 %) als gelbes Öl, anschließend 3',5'-Bis-O-(2-Chlor-2-(2-nitrophenyl)ethoxycarbonyl)thymidin (48 mg) als hellgelben Schaum, dann 3'-O-(2-Chlor-2-(2-nitrophenyl)-ethoxycarbonyl)thymidin (16 mg, 3 %) als farblosen Schaum und zuletzt 5'-O-(2-Chlor-2-(2-nitrophenyl)ethoxycarbonyl)-thymidin (394 mg, 81 %) als farblosen Schaum.
5'-O-(2-Chlor-2-(2-nitrophenyl)ethoxycarbonyl)thymidin:
R_{f} (SiO₂, Toluol/EtOAc/MeOH 5:4:1) 0,34
UV(MeOH), λₘₐₓ [nm] (log ε): 209 (4,34), 262 (4,10)
¹H-NMR (250 MHz, CDCl₃): 8,37 (s, (br), NH); 7,97 (d, J = 8,1, 1 arom. H v. CNPEOC); 7,91 (dd, J = 1,0, 7,9, 1 arom. H v. CNPEOC); 7,72 (t, J = 7,3, 1 arom. H v. CNPEOC); 7,55 (m, 1 arom. H v. CNPEOC); 7,34 (m, H-C(6) v. Thymin), 6,35 (dd, J = 4,0, 6,7, H-C(1'),
   Diastereomer); 6,32 (dd, J = 3,9, 6,5, H-C(1'), Diastereomer); 5,89 (m, β-CH v. CNPEOC); 4,57 (m, α-CH₂ v. CNPEOC), H-C(3'), 2 x H-C(5')); 4,15 (q, J = 3,2, H-C(4')); 2,41 (m, H-C(2')); 2,22 (m, H-C(2')); 1,90 (s, CH₃ v. Thymin, Diastereomer); 1,86 (s, CH₃ v. Thymin, Diastereomer); 1,61 (s (br), OH-C(3'))
Anal. ber. für C₁₉H₂₀ClN₃O₉ (469,834): C 48,57, H 4,29, N 8,94; gef.: C 48,17, H 4,40, N 8,47

### Beispiel 12

### a) 2-Methoxy-2-(2-nitrophenyl)ethanol

Zu einer Lösung von 2-Nitrobenzylmethylether (4,18 g, 25 mmol) in DMSO (3,5 ml, Synthese-Qualität, zusätzlich 2 d über Molekularsieb 4 Å getrocknet) gab man Paraformaldehyd (750 mg, 25 mmol) und DBU (1,85 ml, 12,4 mmol) und ließ bei Raumtemperatur 4 h reagieren. Der pH-Wert wurde mit wenigen Tropfen konz. AcOH von pH 9 auf etwa pH 3,5 eingestellt. Das Gemisch wurde mit CH₂Cl₂ (120 ml) verdünnt und mit H₂O (80 ml) gewaschen. Die wäßrige Phase wurde mit CH₂Cl₂ (2 x 80 ml) nachextrahiert. Die vereinigten organischen Phasen wurden über Na₂SO₄ getrocknet, filtriert und einrotiert. Das Rohprodukt (5,56 g) wurde durch Säulenchromatographie (140 g SiO₂, 21 x 4,2 cm, Lösemittel: Toluol 300 ml, Toluol/EtOAc 20:1 210 ml, 15:1 160 ml, 10:1 220 ml, 15:2 170 ml, 6:1 210 ml, 5:1 180 ml, 4:1 400 ml, 3:1 240 ml, 2:1 240 ml) gereinigt. Zuerst wurde 2-Nitrobenzylmethylether (2,442 g, 58 %) eluiert und dann 2-Methoxy-2-(2-nitrophenyl)ethanol (1,696 g, 34 % bzw. 82 % bezüglich des umgesetzten Edukts).
2-Methoxy-2-(2-nitrophenyl)ethanol (gelber Feststoff):
Rf (SiO₂, CH₂Cl₂/MeOH 100:3) 0,31
Schmelzpunkt: 61 bis 63°C
UV(MeOH), λₘₐₓ [nm] (log ε): 206 (4,09), 256 (3,66)
¹H-NMR (250 MHz, CDCl₃): 8,00 (m, 1 arom. H); 7,70 (m, 2 arom. H); 7,48 (m, 1 arom. H); 4,95 (dd, J = 3,2, 7,2, β-CH); 3,94 (ddd, J = 3,2, 8,8, 11,8, α-CH); 3,67 (ddd, J = 4,4, 7,2, 11,7, α-CH); 3,31 (s, OCH₃); 2,29 (dd, J = 4,4, 8,8, OH)
Anal. ber. für C₉H₁₁NO₄ (197,19): C 54,82, H 5,62, N 7,10; gef.: C 55,14, H 5,57, N 7,15

### b) 2-Methoxy-2-(2-nitrophenyl)ethoxycarbonylchlorid

Zu einer auf 0°C gekühlten Lösung von Diphosgen (1,2 ml, 10 mmol) in THF (15 ml, dest. über CaH₂) wurde in 1 h eine Lösung von 2-Methoxy-2-(2-nitrophenyl)ethanol (986 mg, 5 mmol) und Et₃N (506 mg, 0,693 ml, 5 mmol) in THF (10 ml, dest. über CaH₂) gegeben. Nach weiteren 15 min Rühren bei 0°C wurde das Eisbad entfernt. Man ließ noch 1 h 30 min bei Raumtemperatur rühren. Das Gemisch wurde über Celite abfiltriert und der Niederschlag wurde mit THF (30 ml, dest. über CaH₂) nachgewaschen. Die vereinigten Filtrate wurden einrotiert und im Hochvakuum bei Raumtemperatur getrocknet.

Man erhielt 2-Methoxy-2-(2-nitrophenyl)ethoxycarbonylchlorid (1,264 g, 97 %) als hellbraunes Öl.
R_{f} (SiO₂, CH₂Cl₂) 0,73
UV(MeOH), λₘₐₓ [nm] (log ε): 205 (4,12), 253 (3,71), 348 (Schulter, 2,74)
¹H-NMR (250 MHz, CDCl₃): 8,07 (dd, J = 1,1, 8,2, 1 arom. H); 7,81 (dd, J = 1,6, 7,8, 1 arom. H); 7,73 (dt, J = 1,1, 7,6, 1 arom. H); 7,54 (m, 1 arom. H); 5,13 (dd, J = 3,2, 6,3, β-CH); 4,60 (dd, J = 3,2, 11,2, α-CH); 4,54 (dd, J = 6,4, 11,3, α-CH)
Anal. ber. für C₁₀H₁₀ClNO₅ (259,645): C 46,26, H 3,88, N 5,39; gef.: C 46,74, H 3,88, N 5,40

### c) 5'-0-(2-Methoxy-2-(2-nitrophenyl)ethoxycarbonyl)-thymidin

Thymidin (250 mg, 1,03 mmol) wurde mit Pyridin (2 x 3 ml, pro analysi-Qualität, zusätzlich über Molekularsieb 4 Å getrocknet) koevaporiert, in Pyridin (3,5 ml, s.o.) gelöst und auf ca. -50°C (i-PrOH/N₂) gekühlt. Hierzu wurde in ca. 1,5 h eine Lösung von 2-Methoxy-2-(2-nitrophenyl)ethoxycarbonylchlorid (348 mg, 1,34 mmol) in CH₂Cl₂ (3,5 ml, dest. über CaH₂) gegeben. Man ließ weitere 3,5 h unter i-PrOH/N₂ Kühlung (-40 bis -10°C) rühren. Die Reaktionsmischung wurde mit H₂O (10 ml) verdünnt und mit CH₂Cl₂ (3 x 10 ml) extrahiert. Die organischen Phasen wurden über Na₂SO₄ getrocknet, abfiltriert, einrotiert und mit Toluol (4 x 10 ml) koevaporiert. Das Rohprodukt (578 mg) wurde durch Säulenchromatographie (20 g SiO₂, 12 x 2,1 cm, Lösemittel: CH₂Cl₂ 100 ml, CH₂Cl₂/MeOH 100:1 202 ml, 100:2 102 ml, 100:3 103 ml, 100:4 104 ml, 100:5 105 ml, 100:6 106 ml) gereinigt. Man eluierte zuerst Bis(2-Methoxy-2-(2-nitrophenyl)ethyl)carbonat (88 mg, 16 %) als hellgelben Feststoff und dann eine Mischfraktion von 3'-O-(2-Methoxy-2-(2-nitrophenyl)ethoxycarbonyl)thymidin und 5'-O-(2-Methoxy-2-(2-nitrophenyl)ethoxycarbonyl)thymidin (323 mg). Die Mischfraktion wurde durch erneute Säulenchromatographie (7 g SiO₂, 13 x 1,2 cm, Lösemittel: CH₂Cl₂ 30 ml, CH₂Cl₂/MeOH 100:1 50 ml, 100:2 51 ml, 100:2,5 51 ml, 100:3 51 ml, 100:4 52 ml, 100:5 52 ml) gereinigt. Es wurde zuerst 3'-O-(2-Methoxy-2-(2-nitrophenyl)ethoxycarbonyl)thymidin (11 mg, 2 %) und dann 5'-O-(2-Methoxy-2-(2-nitrophenyl)ethoxycarbonyl)-thymidin (290 mg, 60 %) jeweils als farbloser Schaum eluiert.
5'-O-(2-Methoxy-2-(2-nitrophenyl)ethoxycarbonyl)thymidin:
R_{f} (SiO₂, Toluol/EtOAc/MeOH 5:4:1) 0,40
UV(MeOH), λₘₐₓ [nm] (log ε): 207 (4,33), 263 (4,12)
¹H-NMR (250 MHz, CDCl₃): 8,67 (s (br), NH); 8,03 (d, J = 5,0, 1 arom. H v. MNPEOC); 7,78 (m, 1 arom. H v. MNPEOC); 7,70 (t, J = 7,5, 1 arom. H v. MNPEOC); 7,52 (m, 1 arom. H v. MNPEOC); 7,43 (m, H-C(6) v. Thymin); 6,38 (dd, J = 3,8, 6,7, H-C(1'), Diastereomer); 6,35 (dd, J = 3,7, 6,4, H-C(1'), Diastereomer); 5,14 (m, β-CH v. MNPEOC); 4,42 (m, α-CH₂ v. MNPEOC, H-C(3'), 2 x H-C(5')); 4,15 (q, J = 3,2, H-C(4')); 3,28 (s, OCH₃, Diastereomer); 3,27 (s, OCH₃, Diastereomer); 2,64 (s (br), OH-C(3')); 2,42 (m, H-C(2')); 2,23 (m, H-C(2')); 1,94 (s, CH₃ v. Thymin, Diastereomer); 1,92 (s, CH₃ v. Thymin,
   Diastereomer)
Anal. ber. für C₂₀H₂₃N₃O₁₀ (465,415): C 51,51, H 4,98, N 9,03; gef.: C 51,65, H 5,18, N 8,94

### Beispiel 13

### 5'-O-(2-(2-Chlor-6-nitrophenyl)ethoxycarbonyl)-N⁴-(2-(4-nitrophenyl)ethoxycarbonyl)-2'-desoxycytidin

N⁴-(2-(4-Nitrophenyl)ethoxycarbonyl)-2'-desoxycytidin (5 g, 11,9 mmol) wurde mit Pyridin (2 x 60 ml, pro analysi-Qualität, zusätzlich über Molekularsieb 4 Å getrocknet) koevaporiert, in Pyridin (60 ml, s.o.) gelöst und auf -60°C (i-PrOH/N₂) gekühlt. Hierzu tropfte man in 75 min eine Lösung von 2-(2-Chlor-6-nitrophenyl)-ethoxycarbonylchlorid (4,7 g, 17,8 mmol) in CH₂Cl₂ (60 ml, dest. über CaH₂) und ließ 1 h 45 min unter i-PrOH/N₂ Kühlung (-40 bis -25°C) rühren. Das Gemisch wurde mit CH₂Cl₂ (150 ml) verdünnt und mit H₂O (100 ml) gewaschen. Die wäßrigen Phasen wurden mit CH₂Cl₂ (100 ml) nachextrahiert. Trocknen der organ. Phasen über Na₂SO₄, Filtrieren, Abrotieren des Lösemittels und Koevaporieren mit Toluol (3 x 50 ml) ergaben das Rohprodukt (9,8 g), welches durch Säulenchromatographie (440 g SiO₂, 32 x 5,9 cm, Lösemittel: CH₂Cl₂/MeOH 100:4) gereinigt wurde. Man erhielt 5'-O-(2-(2-Chlor-6-nitrophenyl)ethoxycarbonyl)-N⁴-(2-(4-nitrophenyl)ethoxycarbonyl)-2'-desoxycytidin (5,247 g, 68 %) als farblosen Schaum.
R_{f} (SiO₂, CH₂Cl₂/MeOH 5:1) 0,82
UV(MeOH), λₘₐₓ [nm] (log ε) : 205 (Schulter, 4,56), 211 (4,58), 242 (4,28), 275 (4,17)
¹H-NMR (250 MHz, CDCl₃): 8,18 (m, 2 arom. H v. NPEOC, ortho zu NO₂) : 8,04 (d, H-C(6) v. Cytosin); 8,03 (s (br), NH, teilweise verborgen); 7,73 (dd, 1 arom. H v. CNPEOC); 7,66 (dd, 1 arom. H v. CNPEOC); 7,41 (m, 2 arom. H v. NPEOC, meta zu NO₂); 7,37 (t, H-C(4) v. CNPEOC, teilweise verborgen); 7,22 (d, H-C(5) v. Cytosin), 6,34 (t, H-C(1')); 4,43 (m, H-C(3'), 2 x H-C(5'), α-CH₂ v. CNPEOC, α-CH₂ v. NPEOC); 4,26 (m, H-C(4')); 3,76 (s (br), OH-C(3')); 3,43 (t, β-CH₂ v. CNPEOC); 3,12 (t, β-CH₂ v. NPEOC); 2,73 (m, H-C(2')); 2,14 (m, H-C(2'))
Anal. ber. für C₂₇H₂₆N₅O₁₂Cl (647,981): C 50,05, H 4,04, N 10,81; gef.: C 49,87, H 4,17, N 10,74

### Beispiel 14

### 5'-O-(2-(2-Chlor-6-nitrophenyl)ethoxycarbonyl)-N⁶-(2-(4-nitrophenyl)ethoxycarbonyl)-2'-desoxyadenosin

N⁶-(2-(4-Nitrophenyl)ethoxycarbonyl)-2'-desoxyadenosin (5 g, 11,3 mmol) wurde mit Pyridin (2 x 60 ml, pro analysi-Qualität, zusätzlich über Molekularsieb 4 Å getrocknet) koevaporiert, in Pyridin (60 ml, s.o.) gelöst und auf -60°C (i-PrOH/N₂) gekühlt. Hierzu tropfte man in 2 h eine Lösung von 2-(2-Chlor-6-nitrophenyl)ethoxycarbonylchlorid (4,17 g, 15,8 mmol) in CH₂Cl₂ (60 ml, dest. über CaH₂). Nach weiteren 2 h Rühren unter i-PrOH/N₂ Kühlung (-40 bis -25°C) wurde das Gemisch mit CH₂Cl₂ (150 ml) verdünnt und mit H₂O (100 ml) gewaschen. Die wäßrigen Phasen wurden mit CH₂Cl₂ (100 ml) nachextrahiert. Trocknen der organ. Phasen über Na₂SO₄, Filtrieren, Abrotieren des Lösemittels und Koevaporieren mit Toluol (3 x 50 ml) ergaben das Rohprodukt (8,73 g), welches durch Säulenchromatographie (400 g SiO₂, 28 x 5,7 cm, Lösemittel: CH₂Cl₂/MeOH 100:3) gereinigt wurde. Man erhielt 5'-O-(2-(2-Chlor-6-nitrophenyl)ethoxycarbonyl)-N⁶-(2-(4-nitrophenyl)ethoxycarbonyl)-2'-desoxyadenosin (6,318 g, 83 %) als farblosen Schaum.
R_{f} (SiO₂, Toluol/EtOAc/MeOH 5:4:1) 0,33
UV(MeOH), λₘₐₓ [nm] (log ε): 209 (4,67), 266 (4,46)
¹H-NMR (250 MHz, CDCl₃): 8,71 (s, H-C(8)); 8,53 (s (br), NH, teilweise verborgen), 8,23 (s, H-C(2)); 8,17 (m, 2 arom.
H v. NPEOC, ortho zu NO₂); 7,73 (dd, 1 arom. H v. CNPEOC); 7,65 (dd, 1 arom. H v. CNPEOC); 7,44 (m, 2 arom. H v. NPEOC, meta zu NO₂); 7,36 (t, H-C(4) v. CNPEOC, teilweise verborgen); 6,54 (t, H-C(1')); 4,77 (m, H-C(3')); 4,55 (t, α-CH₂ v. CNPEOC); 4,41 (m, α-CH₂ v. NPEOC, 2 x H-C(5')); 4,29 (q, H-C(4')); 3,43 (t, β-CH₂ v. CNPEOC); 3,16 (t, β-CH₂ v. NPEOC); 3,15 (s (br), OH-C(3'), teilweise verborgen); 2,89 (m, H-C(2')); 2,60 (m, H-C(2'))
Anal. ber. für C₂₈H₂₆N₇O₁₁Cl (672,007): C 50,05, H 3,90, N 14,59; gef.: C 49,62, H 3,96, N 14,33

### Beispiel 15

### 5'-O-(2-(2-Chlor-6-nitrophenyl)ethoxycarbonylthymidin-3'-O-((β-cyanoethyl)(N,N-diisopropylamino)phosphitamid)

In einem mit Argon gespülten Kolben wurden 5'-O-(2-(2-Chlor-6-nitrophenyl)ethoxycarbonyl)thymidin (1,9 g, 4 mmol) und 1H-Tetrazol (140 mg, 2 mmol) in CH₂Cl₂ (20 ml, dest. über CaH₂) und CH₃CN (8 ml, pro analysi-Qualität) suspendiert und mit Bis(diisopropylamino) (β-cyanoethoxy)phosphin (1,87 g, 6,2 mmol) versetzt. Nach 16,5h Rühren bei Raumtemperatur wurde das Gemisch mit CH₂Cl₂ (50 ml) verdünnt und mit gesättigter NaHCO₃-Lsg. (25 ml) gewaschen. Die wäßrige Phase wurde mit CH₂Cl₂ (2 x 25 ml) nachextrahiert. Trocknen der organ. Phasen über Na₂SO₄, Filtrieren und Abrotieren des Lösemittel ergaben das Rohprodukt (3,4 g), welches durch Säulenchromatographie (40 g SiO₂, 12 x 3,1 cm, Lösemittel: Toluol/EtOAc 5:1 160 ml, 4:1 150 ml, 3:1 120 ml, 2:1 150 ml, 1:1 100 ml, 1:2 150 ml, 1:3 160 ml, jeweils unter Zugabe von 1 Vol.-% Et₃N) gereinigt wurde. Man erhielt 5'-O-(2-(2-Chlor-6-nitrophenyl)ethoxycarbonyl)thymidin-3'-O-((β-cyanoethyl)(N,N-diisopropylamino)phosphitamid) (2,012 g, 75 %) als farblosen Schaum.
R_{f} (Toluol/EtOAc/MeOH 5:4:1) 0,64 und 0,70 (Diastereomere)
UV(MeOH), λₘₐₓ [nm] (log ε): 205 (4,45), 262 (4,06)
¹H-NMR (250 MHz, CDCl₃): 8,40 (s, NH); 7,74 (td, 1 arom. H v. CNPEOC; 7,66 (dd, 1 arom. H v. CNPEOC); 7,38 (m, H-C(4) v. CNPEOC); 6,37 (m, H-C(1')); 4,61 bis 4,35 (m, H-C(3'), 2 x H-C(5'), α-CH₂ v. CNPEOC); 4,28 (m, H-C(4'), Diastereomer); 4,22 (m, H-C(4'), Diastereomer); 3,90 - 3,66 (m, α-CH₂ v. Cyanoethoxy); 3,57 (m, 2 x NCH); 3,43 (td, β-CH₂ v. CNPEOC); 2,66 (t, β-CH₂ v. Cyanoethoxy); 2,48 (m, H-C(2')); 2,23 (m, H-C(2')); 1,86 (d, CH3); 1,23 (m, 2 x NC(CH₃)₂)
³¹P-NMR (161,7 MHz, CDCl₃): 149,73 und 149,85 (Diastereomere)
Anal. ber. für C₂₈H₃₇N₅O₁₀PCl (670,056): C 50,19, H 5,57, N 10,45; gef.: C 50,43, H 5,90, N 10,43

### Beispiel 16

### 5'-O-(2-(2-Chlor-6-nitrophenyl)ethoxycarbonyl)-N⁴-(2-(4-nitrophenyl)ethoxycarbonyl)-2'-desoxycytidin-3'-O-((β-cyanoethyl)(N,N-diisopropylamino)phosphitamid)

In einem mit Argon gespülten Kolben wurden 5'-O-(2-(2-Chlor-6-nitrophenyl)ethoxycarbonyl)-N⁴-(2-(4-nitrophenyl)-ethoxycarbonyl)-2'-desoxycytidin (1,9 g, 2,9 mmol) und 1H-Tetrazol (102 mg, 1,45 mmol) in CH₂Cl₂ (20 ml, dest. über CaH₂) und CH₃CN (8 ml, pro analysi-Qualität) mit Bis(diisopropylamino) (β-cyanoethoxy)phosphin (1,32 g, 4,38 mmol) versetzt und bei Raumtemperatur gerührt. Nach 13,5 h wurde die Lösung mit CH₂Cl₂ (50 ml) verdünnt und mit gesättigter NaHCO₃-Lsg. (25 ml) gewaschen. Die wäßrige Phase wurde mit CH₂Cl₂ (2 x 25 ml) nachextrahiert. Die vereinigten organischen Phasen wurden über Na₂SO₄ getrocknet, filtriert und einrotiert. Das Rohprodukt (2,99 g) wurde durch Säulenchromatographie (61 g SiO₂, 18 x 3,2 cm, Lösemittel: PE/Aceton 5:1 170 ml, 4:1 200 ml, 3:1 200 ml, 2:1 600 ml, 3:2 150 ml, 1:1 80 ml, 2:3 300 ml, 1:2 90 ml) gereinigt. Man erhielt 5'-O-(2-(2-Chlor-6-nitrophenyl)ethoxycarbonyl)-N⁴-(2-(4-nitrophenyl)ethoxycarbonyl)-2'-desoxycytidin-3'-O-((β-cyanoethyl)(N,N-diisopropylamino)phosphitamid) (2,29 g, 93 %) als farblosen Schaum.
R_{f} (SiO₂, Toluol/EtOAc/MeOH 5:4:1) 0,61 und 0,67 (Diastereomere)
UV(MeOH), λₘₐₓ [nm] (log ε): 203 (4,66), 209 (Schulter, 4,64), 241 (4,30), 276 (Schulter, 4,18)
¹H-NMR (250 MHz, CDCl₃): 8,18 (m, 2 arom. H v. NPEOC), ortho zu NO₂); 8,02 (m, H-C(6) v. Cytosin) ; 7,74 (td, 1 arom. H v. CNPEOC); 7,66 (dd, 1 arom. H v. CNPEOC); 7,40 (m, 2 arom. H v. NPEOC, meta zu NO₂); 7,39 (m, H-C(4) v. CNPEOC, teilweise verborgen); 7,17 (d, H-C(5) v. Cytosin); 6,28 (m, H-C(1')); 4,47 bis 4,29 (m, α-CH₂ v. NPEOC), α-CH₂ v. CNPEOC), H-C(3'), H-C(4'), 2 x H-C(5')); 3,90 bis 3,53 (m, α-CH₂ v. Cyanoethoxy, 2 x NCH); 3,43 (t, β-CH₂ v. CNPEOC); 3,12 (t, β-CH₂ v. NPEOC); 2,73 (m, H-C(2') teilweise verborgen); 2,65 (t, β-CH₂ v. Cyanoethoxy); 2,17 (m, H-C(2')); 1,23 (m, 2 x NC(CH₃)₂)
³¹P-NMR (161,7 MHz, CDCl₃): 149,67 und 150,02 (Diastereomere)
Anal. ber. für C₃₆H₄₃N₇O₁₃PCl (848,203): C 50,98, H 5,11, N 11,56; gef.: C 50,88, H 5,18, N 11,36

### Beispiel 17

### 5'-O-(2-(2-Chlor-6-nitrophenyl)ethoxycarbonyl)-N⁶-(2-(4-nitrophenyl)ethoxycarbonyl)-2'-desoxyadenosin-3'-O-((β-cyanoethyl)(N,N-diisopropylamino)phosphitamid)

In einem mit Argon gespülten Kolben wurde ein Gemisch von 5'-O-(2-(2-Chlor-6-nitrophenyl)ethoxycarbonyl)-N⁶-(2-(4-nitrophenyl)ethoxycarbonyl)-2'-desoxyadenosin (2 g, 2,98 mmol) und 1H-Tetrazol (104 mg, 1,49 mmol) in CH₂Cl₂ (20 ml, dest. über CaH₂) und CH₃CN (8 ml, pro analysi-Qualität) mit Bis(diisopropylamino) (β-cyanoethoxy)phosphin (1,36 g, 4,51 mmol) versetzt und bei Raumtemperatur 13,5 h gerührt. Die Lösung wurde mit CH₂Cl₂ (50 ml) verdünnt und mit gesättigter NaHCO₃-Lsg. (25 ml) gewaschen. Die wäßrige Phase wurde mit CH₂Cl₂ (2 x 25 ml) nachextrahiert. Die vereinigten organischen Phasen wurden über Na₂SO₄ getrocknet, filtriert und einrotiert. Das Rohprodukt wurde durch Säulenchromatographie (92 g SiO₂, 13 x 4,2 cm, Lösemittel: PE/Aceton 6:1 140 ml, 5:1 180 ml, 4:1 200 ml, 3:1 240 ml, 2:1 750 ml, 3:2 150 ml, 1:1 400 ml, 2:3 600 ml, jeweils unter Zugabe von 1 Vol.-% Et₃N) gereinigt. Man erhielt 5'-O-(2-(2-Chlor-6-nitrophenyl)ethoxycarbonyl)-N⁶-(2-(4-nitrophenyl)ethoxycarbonyl)-2'-desoxyadenosin-3'-O-((β-cyanoethyl)(N,N-diisopropylamino)phosphitamid) (2,146 g, 83 %) als farblosen Schaum.
R_{f} (SiO₂, Toluol/EtOAc/MeOH 5:4:1) 0,71 und 0,76 (Diastereomere)
UV(MeOH), λₘₐₓ [nm] (log ε): 205 (4,68), 266 (4,43)
¹H-NMR (250 MHz, CDCl₃): 8,74 (d, H-C(8) v. Adenin); 8,23 (s, H-C(2) v. Adenin); 8,18 (m, 2 arom. H v. NPEOC, ortho zu NO₂); 7,73 (m, 1 arom. H v. CNPEOC); 7,65 (m, 1 arom. H v. CNPEOC); 7,44 (m, 2 arom. H v. NPEOC, meta zu NO₂); 7,36 (t, H-C(4) v. CNPEOC); 6,52 (m, H-C(1')); 4,77 (m, H-C(3')); 4,54 (t, α-CH₂ v. CNPEOC); 4,39 (m, α-CH₂ v. NPEOC, H-C(4'), 2 x H-C(5')); 3,93 bis 3,59 (m, 2 x NCH, α-CH₂ v. Cyanoethoxy); 3,41 (td, β-CH₂ v. CNPEOC); 3,16 (t, β-CH₂ v. NPEOC); 2,90 (m, H-C(2')); 2,71 (m, H-C(2'), teilweise verborgen); 2,67 (m, β-CH₂ v. Cyanoethoxy); 1,24 (m, 2 x NC(CH₃)₂)
³¹P-NMR (161,7 MHz, CDCl₃): 149,70 und 149,79 (Diastereomere)
Anal. ber. für C₃₇H₄₃N₉O₁₂PCl (872,229): C 50,95, H 4,97, N 14,45; gef.: C 50,92, H 5,11, N 14,21

Zusammenfassung der Herstellungsbeispiele

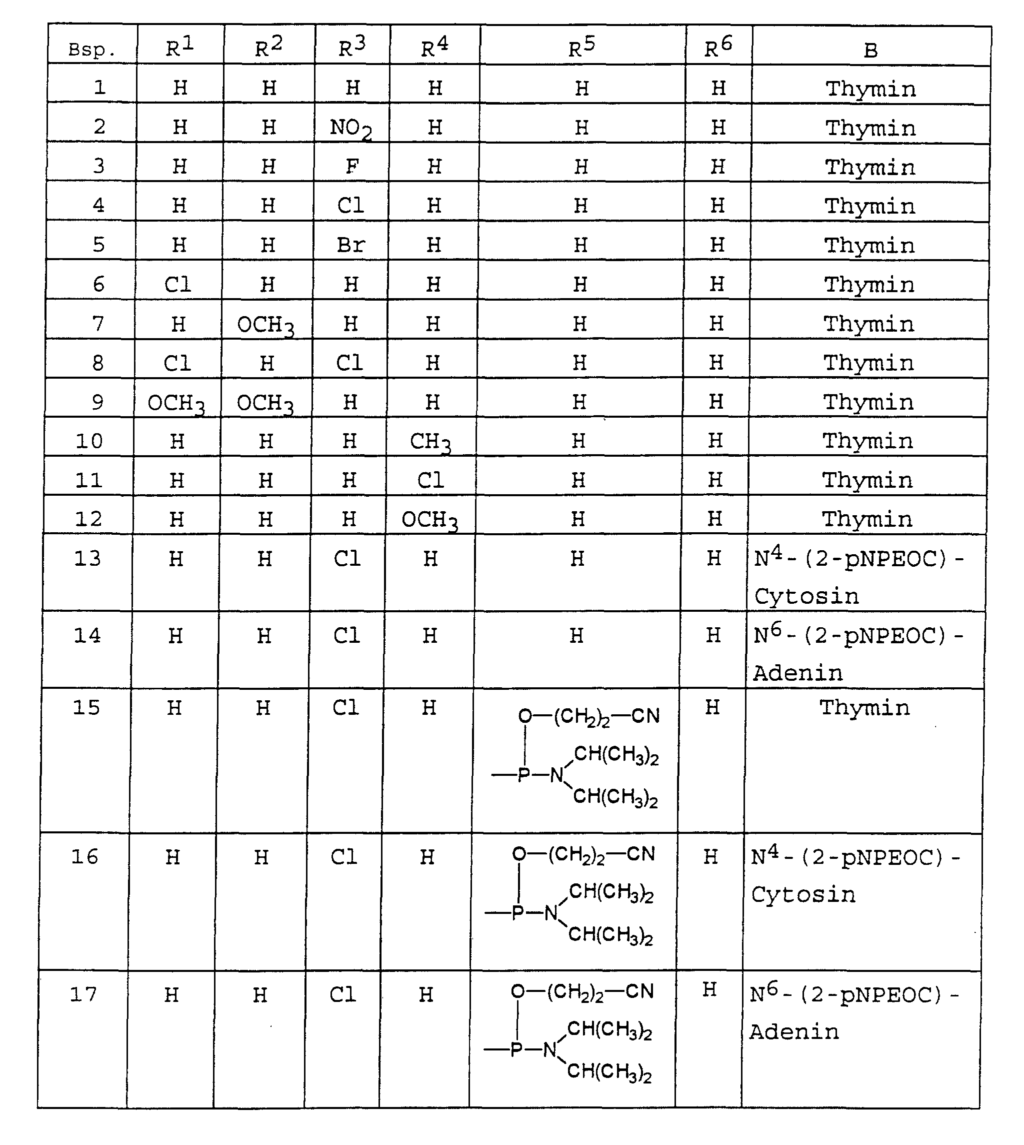

### Bestrahlungsexperimente

### 1. Durchführung

Die entsprechend geschützten Thymidin-Nucleoside wurden mittels einer Bestrahlungsapparatur bestrahlt, die aus einer Hg-Höchstdrucklampe (OSRAM HBO, 200 W), einer Sammellinse, einem Verschluß mit einem elektronischen Steuergerät zur Einstellung der Verschlußzeiten sowie einem temperierbaren Küvettenhalter bestand. Es war außerdem ein Wärmefilter (0,032 molare CuSO₄-Lsg.) zwischen Lampe und Probe eingebaut. Es wurden 0,2 mM Lösungen der Nucleoside in MeOH/H₂O 1:1 bei 20 bis 30°C mit dem gesamten Lampenspektrum (polychromatisches Licht mit Wellenlängen λ > 289 nm) belichtet.

Die Abspaltung der Schutzgruppen wurde durch HPLC quantitativ verfolgt. Die HPLC-Anlage bestand aus folgenden Geräten: Merck-Hitachi L-6200 Intelligent Pump, Merck-Hitachi Intelligent Auto Sampler AS 4000, Merck-Hitachi Interface, Merck Lichrosorb-Säule RP 18, 125 x 5 mm. Das UV/VIS-Spektrophotometer war ein UVIKON 730 LC der Fa. Kontron. Die Detektion erfolgte bei 260 nm. Die Integration der Chromatogramm-Signale erfolgte mittels Software der Fa. Merck: D-6000 HPLC-Manager.

Es wurde grundsätzlich in MeOH/H₂O-Gemischen chromatographiert. Dabei wurde folgender Gradient verwendet (Flow: 1 ml/min):

| Zeit (min) | MeOH/H₂O 1:1 | H₂O | MeOH |
|---|---|---|---|
| 0 | 10 | 90 | 0 |
| 5 | 10 | 90 | 0 |
| 15 | 90 | 10 | 0 |
| 30 | 50 | 0 | 50 |
| 35 | 10 | 90 | 0 |

Für Thymidin und die geschützten Nucleoside wurden durch Einspritzen von Verdünnungsreihen in den Chromatographen Eichkurven erstellt.

Aus den belichteten Lösungen wurden nach bestimmten Zeitintervallen Proben (Schleifen-Volumen: 20 µl) entnommen. Jede Probe wurde zweimal in den Chromatographen eingespritzt. Für die weitere Auswertung wurden die Mittelwerte dieser Doppelbestimmungen genommen. Die Peakflächen der Chromatogramme wurden mittels der oben erstellten Eichkurven in die Konzentrationen der geschützten Nucleoside bzw. von Thymidin umgerechnet. Die so erhaltenen Konzentrationswerte wurden durch die maximal erreichbare Konzentration von 0,2 mM (= Konzentration der geschützten Nucleoside zu Beginn der Belichtung) dividiert und als "Relative Menge" in Prozent gegen die Bestrahlungszeit aufgetragen. Die in Abschnitt 2.3 angegebene Tabelle zeigt ein Resultat einer solchen Messung mit der Verbindung 5'-O-(2-(2-Chlor-6-nitrophenyl)-ethoxycarbonyl)thymidin.

### 2. Beispiele

### 2.1 Eichung Thymidin

Für die Eichung von Thymidin wurde eine Verdünnungsreihe mit folgenden Konzentrationen erstellt: 0,2 mM, 0,16 mM, 0,12 mM, 0,08 mM und 0,04 mM. Hiervon wurden je Konzentration drei Einspritzungen in den Chromatographen gemacht. Aus den resultierenden Mittelwerten sowie dem Nullpunktswert (0 Peakfläche = 0 Konzentration) wurden mittels linearer Regression die Eichgerade berechnet (siehe Tabelle).

| Eichung Thymidin | |
|---|---|
| Peakfläche | Konzentration Thymidin (mM) |
| 516,893 | 0,20 |
| 414,730 | 0,16 |
| 307,575 | 0,12 |
| 208,952 | 0,08 |
| 99,5080 | 0,04 |
| 0,00000 | 0,00 |

Lineare Regression ergab: y = 4,96777·10⁻⁴ + 3,85757·10⁻⁷·x mit x = Peakfläche und y = Konzentration Thymidin; r = 0,9999.

### 2.2 Eichung 5'-O-(2-(2-Chlor-6-nitrophenyl)-ethoxycarbonyl)thymidin

Es wurde eine Verdünnungsreihe mit folgenden drei Konzentrationen erstellt: 0,2 mM, 0,14 mM und 0,08 mM. Die Mittelwerte aus drei Einspritzungen je Konzentration sowie der Nullpunktswert wurden zur Berechnung der Regressionsgerade eingesetzt (siehe Tabelle):

| Eichung CNPEOC-T | |
|---|---|
| Peakfläche | Konzentration CNPEOC-T (mM) |
| 617,057 | 0,20 |
| 435,070 | 0,14 |
| 238,624 | 0,08 |
| 0,00000 | 0,00 |

Lineare Regression ergab: y = 9,27999·10⁻⁴ + 3,22516·10⁻⁷·x mit x = Peakfläche und y = Konzentration CNPEOC-T; r = 0,9998.

### 2.3 Ergebnisse

### (a) 5'-O-(2-(2-Chlor-6-nitrophenyl)-ethoxycarbonyl)thymidin

| Bestrahlung von 5'-O-(2-(2-Chlor-6- nitrophenyl)ethoxycarbonyl)thymidin | | | |
|---|---|---|---|
| Bestrahlungszeit (min) | Peakfläche CNPEOC-T | Konzentration CNPEOC-T (mM) | Relative Menge CNPEOC-T (%) |
| 1,00000 | 458911 | 0,1489 | 74,5 |
| 2,00000 | 253292 | 0,0826 | 41,3 |
| 3,00000 | 250989 | 0,0819 | 40,9 |
| 5,00000 | 84081,0 | 0,0281 | 14,0 |
| 15,0000 | 24181,0 | 0,0088 | 4,4 |
| 30,0000 | 0 | 0 | 0 |
| 60,0000 | 0 | 0 | 0 |
| 120,000 | 0 | 0 | 0 |

| Bestrahlungszeit (min) | Peakfläche Thymidin | Konzentration Thymidin (mM) | Relative Menge Thymidin (%) |
|---|---|---|---|
| 1,00000 | 83460,0 | 0,0327 | 16,3 |
| 2,00000 | 221149 | 0,0858 | 42,9 |
| 3,00000 | 228893 | 0,0888 | 44,4 |
| 5,00000 | 330106 | 0,1278 | 63,9 |
| 15,0000 | 432514 | 0,1673 | 83,7 |
| 30,0000 | 437588 | 0,1693 | 84,6 |
| 60,0000 | 442230 | 0,1711 | 85,5 |
| 120,000 | 407543 | 0,1577 | 78,9 |

Diese Tabelle zeigt deutlich, daß die Photolyse über einen sehr großen Zeitraum der Kinetik einer ersten Ordnung folgt, was auf einen eindeutigen Abspaltungsmechanismus ohne ausbeutemindernde Nebenreaktionen schließen läßt.

### (b) Weitere erfindungsgemäße Verbindungen

Die nach der oben angegebenen Methode erhaltenen und bezüglich Halbwertszeit und Ausbeute (Prozentsatz entschütztes Nucleosid-Derivat, bezogen auf die maximal erreichbare Konzentration) ausgewerteten Ergebnisse einiger weiterer erfindungsgemäßen Derivate und der zwei Vergleichsverbindungen V1 (5'-O-(2-Nitrobenzyloxycarbonyl)-thymidin) und V2 (5'-O-(2,4-Dinitrobenzyloxycarbonyl)-thymidin) werden in der nachstehenden Tabelle zusammengefaßt:

| Verbindung Nr. | t_{0,5} (min) | Ausbeute (% Cₘₐₓ) |
|---|---|---|
| 1 | 2,6 | 79 |
| 2 | 1,37 | 92 |
| 4 | 1,71 | 86 |
| 5 | 1,71 | 89 |
| 6 | 2,3 | 70 |
| 8 | 1,68 | 77 |
| 9 | 7,2 | 77 |
| 10 | 0,7 | 89 |
| 11 | 1,46 | 97 |
| V1 | 2,5 | 55 |
| V2 | 3,3 | 38 |

Wie aus der Tabelle ersichtlich ist, sind die erfindungsgemäßen Nucleosid-Derivate hinsichtlich der photolytischen Abspaltung der 5'-Schutzgruppe in Bezug auf Schnelligkeit und hohe Ausbeuten (vgl. insbesondere Verbindungen 10 und 11) den Schutzgruppen entsprechend dem Stand der Technik (vgl. V1 und V2) deutlich überlegen.

### 2.4 Anwendungsbeispiel

Die Verbindung 5'-0-(2-(2-Chlor-6-nitrophenyl)-ethoxycarbonyl)thymidin und andere erfindungsgemäße Nucleosid-Derivate wurden nach einer Methodik gemäß S.P.A. Fodor et al. Science 1991, 251, S.767ff zur Darstellung von Oligonucleotiden auf einem DNA-Chip eingesetzt. Es zeigte sich, daß die erfindungsgemäßen Verbindungen eine unkomplizierte Oligonucleotidsynthese mit sehr hohen Ausbeuten erlaubten, so daß sie in der Praxis für lichtgesteuerte Parallel-Synthesen von Oligonucleotiden geeignet sind.

## Patentansprüche

1. Nucleosid-Derivate mit photolabilen Schutzgruppen der allgemeinen Formel (I) 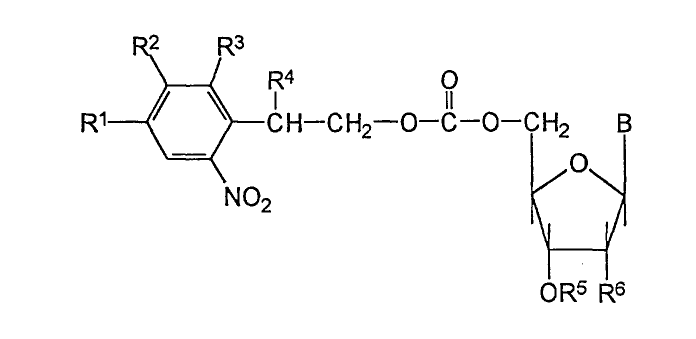 in der
R¹ = H, CN, OCH₃, Halogen oder Alkyl oder Alkoxyalkyl mit 1 bis 4 C-Atomen
R² = H, OCH₃
R³ = H, F, Cl, Br, NO₂
R⁴ = H, Halogen, OCH₃ oder ein Alkylrest mit 1 bis 4 C-Atomen
R⁵ = H oder eine übliche funktionelle Gruppe zur Herstellung von Oligonucleotiden
R⁶ = H, OH, Halogen oder XR⁸, wobei X = O oder S und R⁸ eine in der Nucleotidchemie übliche Schutzgruppe darstellt,
B = Adenin, Cytosin, Guanin, Thymin, Uracil, 2,6-Diaminopurin-9-yl, Hypoxanthin-9-yl, 5-Methylcytosin-1-yl,
5-Amino-4-imidazolcarbonsäureamid-1-yl oder
5-Amino-4-imidazolcarbonsäureamid-3-yl, wobei im Falle von B = Adenin, Cytosin oder Guanin die primäre Aminofunktion ggf. eine permanente Schutzgruppe aufweist.

2. Nucleosid-Derivate nach Anspruch 1, **dadurch gekennzeichnet, daß** im Falle von R⁴ ≠ H R¹ = R² = R³ = H ist.

3. Nucleosid-Derivate nach Anspruch 1, **dadurch gekennzeichnet, daß** im Falle von R² = OCH₃ R³ = H ist.

4. Nucleosid-Derivate nach den Ansprüchen 1 bis 3, **dadurch gekennzeichnet, daß** R⁴ einen Methylrest darstellt.

5. Nucleosid-Derivate nach den Ansprüchen 1 bis 4, **dadurch gekennzeichnet, daß** R⁵ eine Phosphitamidgruppe der Formel: 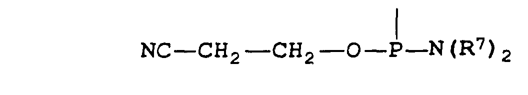 oder 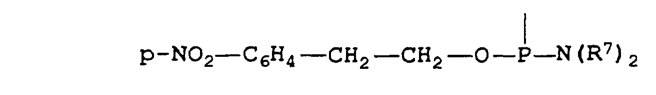 darstellt,
wobei die R⁷-Gruppen gleich oder verschieden sind und lineare oder verzweigte Alkylreste mit 1 bis 4 C-Atomen bedeuten.

6. Nucleosid-Derivate nach Anspruch 5, **dadurch gekennzeichnet, daß** R⁷ einen Ethyl- oder Isopropylrest darstellt.

7. Nucleosid-Derivate nach den Ansprüchen 1 bis 6, **dadurch gekennzeichnet, daß** R⁶ eine Gruppe XR⁸ ist und R⁸ im Fall von X=O eine Alkyl-, Alkenyl-, Acetal- oder Silylether-Schutzgruppe oder im Fall von X=S eine Alkyl-Schutzgruppe darstellt.

8. Nucleosid-Derivate nach Anspruch 7, **dadurch gekennzeichnet, daß** als R⁶ ein O-Methyl- oder O-Ethylrest, ein O-Allylrest, ein O-Tetrahydropyranylbzw. O-Methoxytetrahydropyranyl-Rest oder ein O-t-Butyldimethylsilyl-Rest eingesetzt wird.

9. Nucleosid-Derivate nach den Ansprüchen 1 bis 8, **dadurch gekennzeichnet, daß** man im Falle von B = Adenin, Cytosin oder Guanin als permanente Schutzgruppe Phenoxyacetyloder Dimethylformamidino-Reste einsetzt.

10. Nucleosid-Derivate nach den Ansprüchen 1 bis 8, **dadurch gekennzeichnet, daß** man im Falle von B = Adenin als permanente Schutzgruppe Benzoyl- oder p-Nitrophenylethoxycarbonyl-(p-NPEOC)-Reste verwendet.

11. Nucleosid-Derivate nach den Ansprüchen 1 bis 8, **dadurch gekennzeichnet, daß** im Falle von B = Guanin die permanente Schutzgruppe Isobutyroyl- oder p-NPEOC-Reste darstellen.

12. Nucleosid-Derivate nach den Ansprüchen 1 bis 8, **dadurch gekennzeichnet, daß** man im Falle von B = Cytosin als permanente Schutzgruppen Benzoyl- oder p-NPEOC-Reste einsetzt.

13. Nucleosid-Derivate nach den Ansprüchen 1 bis 12, **dadurch gekennzeichnet, daß** als Halogenatom in R¹ oder R⁶ ein Fluor-, Chlor- oder Bromatom eingesetzt wird.

14. Verfahren zur Herstellung von Nucleosid-Derivaten nach den Ansprüchen 1 bis 13 in mindestens zwei Stufen, **dadurch gekennzeichnet, daß** man
a) einen Alkohol der allgemeinen Formel (II) 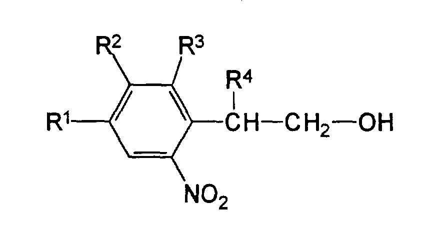 in der R¹, R², R³, R⁴ die oben angegebene Bedeutung besitzen, mit einem Phosgen-Derivat umsetzt und anschließend
b) den in Stufe a) gebildeten Chlorkohlensäureester mit Nucleosiden der allgemeinen Formel (III) 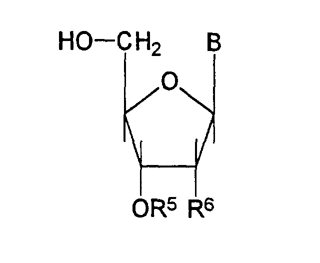 in der R⁵, R⁶ und B die oben angegebene Bedeutung besitzen, reagieren läßt und ggf. anschließend
c) in der 3'-Stellung der Nucleosid-Derivate mit R⁵ = H die Phosphitamid-Gruppe 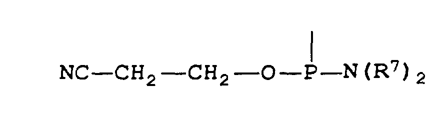 oder 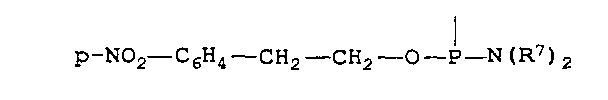 nach bekannten Methoden einführt.

15. Verfahren nach Anspruch 14, **dadurch gekennzeichnet, daß** man Stufe a) in einem unpolaren organischen Lösemittel bei Temperaturen zwischen -20 und +25°C durchführt.

16. Verfahren nach den Ansprüchen 14 und 15, **dadurch gekennzeichnet, daß** man in Stufe a) das Phosgenderivat in zwei- bis fünffachem Überschuß bezogen auf die Alkoholkomponente einsetzt.

17. Verfahren nach den Ansprüchen 14 bis 16, **dadurch gekennzeichnet, daß** man in Stufe a) als unpolares organisches Lösemittel Toluol oder THF verwendet.

18. Verfahren nach den Ansprüchen 14 bis 17, **dadurch gekennzeichnet, daß** die Konzentration der Alkoholkomponente in Stufe a) 0,1 bis 10,0 mmol pro 10 ml Solvens beträgt.

19. Verfahren nach den Ansprüchen 14 bis 18, **dadurch gekennzeichnet, daß** man Stufe b) in einem Lösungsmittelgemisch bestehend aus Dichlormethan und einem polaren organischen Lösemittel ggf. in Gegenwart einer Base bei Temperaturen zwischen -60 und +25°C durchführt.

20. Verfahren nach den Ansprüchen 14 bis 19, **dadurch gekennzeichnet, daß** man in Stufe b) als polares organisches Lösemittel DMF oder Pyridin heranzieht.

21. Verfahren nach den Ansprüchen 14 bis 20, **dadurch gekennzeichnet, daß** man in Stufe b) ein Lösemittelgemisch bestehend aus Dichlormethan und DMF sowie eine Base ausgewählt aus der Gruppe Pyridin, Triethylamin und Ethyldiisopropylamin einsetzt.

22. Verfahren nach den Ansprüchen 20 bis 21, **dadurch gekennzeichnet, daß** das Mischungsverhältnis Dichlormethan zu Pyridin bzw. DMF 1:1 bis 3:1 beträgt.

23. Verfahren nach den Ansprüchen 14 bis 22, **dadurch gekennzeichnet, daß** das Molverhältnis Nucleosid zu Chlorkohlensäureester 1:1 bis 1:2 beträgt.

24. Verfahren nach den Ansprüchen 14 bis 23, **dadurch gekennzeichnet, daß** man in Stufe b) das in Pyridin oder DMF/Base gelöste Nucleosid vorlegt und eine Lösung des Chlorkohlensäureesters in Dichlormethan bei der jeweiligen Reaktionstemperatur zutropfen läßt.

25. Verfahren nach den Ansprüchen 14 bis 24, **dadurch gekennzeichnet, daß** die Konzentration des Nucleosids im Lösemittelgemisch in Stufe b) 0,1 bis 3,0 mmol pro 10 ml Solvens beträgt.

26. Verfahren nach den Ansprüchen 14 bis 25, **dadurch gekennzeichnet, daß** man die Einführung der Phosphitamid-Gruppe (Stufe c) durch Umsetzung der Nucleosid-Derivate (mit R⁵ = H) mit den entsprechenden Phosphinen in Gegenwart von 1H-Tetrazol als Aktivator in einem Lösemittelgemisch bestehend aus Dichlormethan und Acetonitril bei Temperaturen zwischen 0 und 25°C vornimmt.

27. Verwendung eines Nucleosid-Derivats der allgemeinen Formel (I) gemäß Anspruch 1 für die lichtgesteuerte Synthese von Oligonucleotiden.

28. Verwendung gemäß Anspruch 27, **dadurch gekennzeichnet, daß** die lichtgesteuerte Oligonucleotidsynthese auf einem festen Trägermaterial erfolgt.

29. Verwendung gemäß Anspruch 27 oder 28, **dadurch gekennzeichnet, daß** das Nucleosid-Derivat wie in einem oder mehreren der Ansprüche 2 bis 13 definiert ist.

## Claims

1. Nucleoside derivatives having photolabile protective groups of the general formula (I) 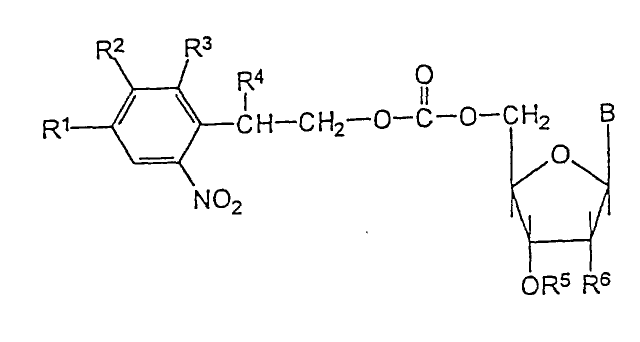 in which
R¹ = H, CN, OCH₃, halogen or alkyl or alkoxyalkyl having 1 to 4 C atoms
R² = H, OCH₃
R³ = H, F, Cl, Br, NO₂
R⁴ = H, halogen, OCH₃ or an alkyl radical having 1 to 4 C atoms
R⁵ = H or a conventional functional group for producing oligonucleotides
R⁶ = H, OH, halogen or XR⁸, wherein X = O or S and R⁸ represents a protective group which is conventional in nucleotide chemistry,
B = adenine, cytosine, guanine, thymine, uracil, 2,6-diaminopurin-9-yl, hypoxanthin-9-yl, 5-methylcytosin-1-yl, 5-amino-4-imidazolcarboxylic acid amid-1-yl or 5-amino-4-imidazolcarboxylic acid amid-3-yl, wherein in the case of B = adenine, cytosine or guanine, the primary amino function optionally has a permanent protective group.

2. Nucleoside derivatives according to claim 1, **characterised in that** in the case of R⁴≠ H, R¹ = R² = R³ = H.

3. Nucleoside derivatives according to claim 1, **characterised in that** in the case of R² = OCH₃, R³ = H.

4. Nucleoside derivatives according to claims 1 to 3, **characterised in that** R⁴ represents a methyl radical.

5. Nucleoside derivatives according to claims 1 to 4, **characterised in that** R⁵ represents a phosphite amide group of the formula: 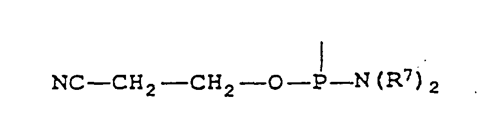 or 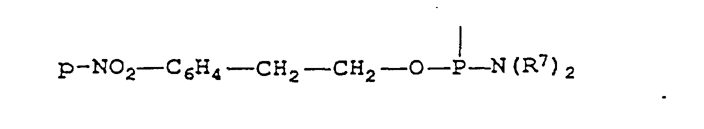 wherein the R⁷ groups are the same or different and denote linear or branched alkyl radicals having 1 to 4 C atoms.

6. Nucleoside derivatives according to claim 5, **characterised in that** R⁷ represents an ethyl or isopropyl radical.

7. Nucleoside derivatives according to claims 1 to 6, **characterised in that** R⁶ is a group XR⁸ and R⁸ in the case of X = O represents an alkyl, alkenyl, acetal or silyl ether protective group or in the case of X = S represents an alkyl protective group.

8. Nucleoside derivatives according to claim 7, **characterised in that** an O-methyl or O-ethyl radical, an O-allyl radical, an O-tetrahydropyranyl or O-methoxytetrahydropyranyl radical or an O-t-butyl dimethylsilyl radical is used as R⁶.

9. Nucleoside derivatives according to claims 1 to 8, **characterised in that** in the case of B = adenine, cytosine or guanine, phenoxyacetyl or dimethylformamidino radicals are used as permanent protective group.

10. Nucleoside derivatives according to claims 1 to 8, **characterised in that** in the case of B = adenine, benzoyl or p-nitrophenylethoxycarbonyl-(p-NPEOC) radicals are used as permanent protective group.

11. Nucleoside derivatives according to claims 1 to 8, **characterised in that** in the case of B = guanine, isobutyroyl or p-NPEOC radicals represent the permanent protective group.

12. Nucleoside derivatives according to claims 1 to 8, **characterised in that** in the case of B = cytosine, benzoyl or p-NPEOC radicals are used as permanent protective groups.

13. Nucleoside derivatives according to claims 1 to 12, **characterised in that** a fluorine, chlorine or bromine atom is used as halogen atom in R¹ or R⁶.

14. Process for producing nucleoside derivatives according to claims 1 to 13 in at least two stages, **characterised in that**
a) an alcohol of the general formula (II) 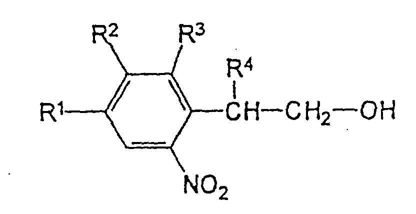 in which R¹, R², R³, R⁴ have the meaning given above, is reacted with a phosgene derivative, and then
b) the chlorocarbonate formed in stage a) is allowed to react with nucleosides of the general formula (III) 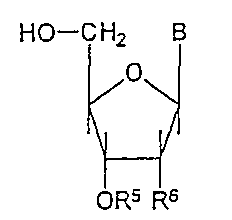 in which R⁵, R⁶ and B have the meaning given above, and optionally then
c) in the 3' position of the nucleoside derivatives where R⁵ = H, the phosphite amide group 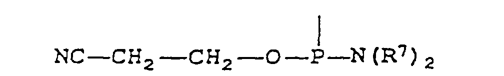 or 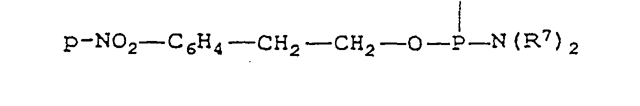 is introduced by known methods.

15. Process according to claim 14, **characterised in that** stage a) is carried out in a non-polar organic solvent at temperatures between -20 and +25°C.

16. Process according to claims 14 and 15, **characterised in that** in stage a) the phosgene derivative is used in two to five times excess based on the alcohol component.

17. Process according to claims 14 to 16, **characterised in that** in stage a) toluene or THF is used as non-polar organic solvent.

18. Process according to claims 14 to 17, **characterised in that** the concentration of the alcohol component in stage a) is 0.1 to 10.0 mmoles per 10 ml of solvent.

19. Process according to claims 14 to 18, **characterised in that** stage b) is carried out in a solvent mixture consisting of dichloromethane and a polar organic solvent, optionally in the presence of a base at temperatures between -60 and +25°C.

20. Process according to claims 14 to 19, **characterised in that** in stage b) DMF or pyridine is used as polar organic solvent.

21. Process according to claims 14 to 20, **characterised in that** in stage b) a solvent mixture consisting of dichloromethane and DMF is used as well as a base selected from the group pyridine, triethylamine and ethyldiisopropylamine.

22. Process according to claims 20 to 21, **characterised in that** the mixing ratio of dichloromethane to pyridine or DMF is 1:1 to 3:1.

23. Process according to claims 14 to 22, **characterised in that** the molar ratio of nucleoside to chlorocarbonate is 1:1 to 1:2.

24. Process according to claims 14 to 23, **characterised in that** in stage b) the nucleoside dissolved in pyridine or DMF/base is initially provided and a solution of the chlorocarbonate in dichloromethane is allowed to be added dropwise at the particular reaction temperature.

25. Process according to claims 14 to 24, **characterised in that** the concentration of the nucleoside in the solvent mixture in stage b) is 0.1 to 3.0 mmoles per 10 ml of solvent.

26. Process according to claims 14 to 25, **characterised in that** the introduction of the phosphite amide group (stage c) is carried out by reacting the nucleoside derivatives (where R⁵ = H) with the corresponding phosphines in the presence of 1H-tetrazole as activator in a solvent mixture consisting of dichloromethane and acetonitrile at temperatures between 0 and 25°C.

27. Use of a nucleoside derivative of the general formula (I) according to claim 1 for the light-controlled synthesis of oligonucleotides.

28. Use according to claim 27, **characterised in that** the light-controlled oligonucleotide synthesis takes place on a solid carrier material.

29. Use according to claim 27 or 28, **characterised in that** the nucleoside derivative is defined as in one or more of claims 2 to 13.

## Revendications

1. Dérivés de nucléosides à groupes protecteurs photolabiles de formule générale (I) 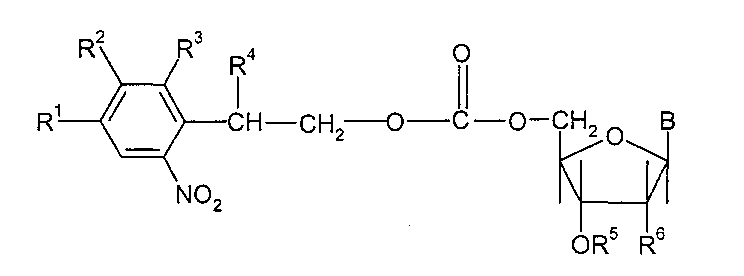 où
R¹ = H, CN, OCH₃, halogène ou alkyle ou alcoxyalkyle de 1 à 4 atomes de carbone
R² = H, OCH₃
R³ = H, F, Cl, Br, NO₂
R⁴ = H, halogène, OCH₃ ou un reste alkyle de 1 à 4 atomes de carbone
R⁵ = H ou un groupe fonctionnel courant pour la préparation d'oligonucléotides
R⁶ = H, OH, halogène ou XR⁸, où X = O ou S et R⁸ représente un groupe protecteur courant en chimie des nucléotides
B = adénine, cytosine, guanine, thymine, uracile, 2,6-diaminopurin-9-yle, hypoxanthin-9-yle, 5-méthylcytosin-1-yle, 5-amino-4-imidazolcarboxamid-1-yle ou 5-amino-4-imidazolcarboxamid-3-yle, où dans le cas où B = adénine, cytosine ou guanine, la fonction amino primaire comporte éventuellement un groupe protecteur permanent.

2. Dérivés de nucléosides selon la revendication 1 **caractérisés en ce que**, dans le cas où R⁴ ≠ H, R¹ = R² = R³ = H.

3. Dérivés de nucléosides selon la revendication 1 **caractérisés en ce que**, dans le cas où R² = OCH₃, R³ = H.

4. Dérivés de nucléosides selon les revendications 1 à 3 **caractérisés en ce que** R⁴ représente un reste méthyle.

5. Dérivés de nucléosides selon les revendications 1 à 4 **caractérisés en ce que** R⁵ représente un groupe phosphitamide de formule 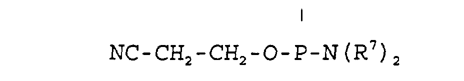 ou 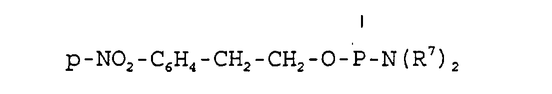 où les groupes R⁷ sont identiques ou différents et représentent des restes alkyle linéaires ou ramifiés de 1 à 4 atomes de carbone.

6. Dérivés de nucléosides selon la revendication 5 **caractérisés en ce que** R⁷ représente un reste éthyle ou isopropyle.

7. Dérivés de nucléosides selon les revendications 1 à 6 **caractérisés en ce que** R⁶ est un groupe XR⁸ et R⁸, dans le cas où X = O, représente un groupe protecteur alkyle, alcényle, acétal ou silyléther ou, dans le cas où X = S, un groupe protecteur alkyle.

8. Dérivés de nucléosides selon la revendication 7 **caractérisés en ce qu'**un reste O-méthyle ou O-éthyle, un reste O-allyle, un reste O-tétrahydropyranyle ou O-méthoxytétrahydropyranyle ou un reste O-t-butyldiméthylsilyle est utilisé comme R⁶.

9. Dérivés de nucléosides selon les revendications 1 à 8 **caractérisés en ce que**, dans le cas où B = adénine, cytosine ou guanine, on utilise des restes phénoxyacétyle ou diméthylformamidino comme groupe protecteur permanent.

10. Dérivés de nucléosides selon les revendications 1 à 8 **caractérisés en ce que**, dans le cas où B = adénine, on utilise des restes benzoyle ou p-nitrophényléthoxycarbonyle (p-NPEOC) comme groupe protecteur permanent.

11. Dérivés de nucléosides selon les revendications 1 à 8 **caractérisés en ce que**, dans le cas où B = guanine, le groupe protecteur permanent représente des restes isobutyryle ou p-NPEOC.

12. Dérivés de nucléosides selon les revendications 1 à 8 **caractérisés en ce que**, dans le cas où B = cytosine, on utilise des restes benzoyle ou p-NPEOC comme groupes protecteurs permanents.

13. Dérivés de nucléosides selon les revendications 1 à 12 **caractérisés en ce qu'**un atome de fluor, de chlore ou de brome est utilisé comme atome d'halogène dans R¹ ou R⁶.

14. Procédé de préparation de dérivés de nucléosides selon les revendications 1 à 13 en au moins deux étapes, **caractérisé en ce que** l'on fait réagir
a) un alcool de formule générale (II) 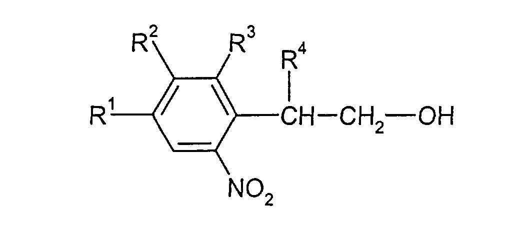 où R¹, R², R³, R⁴ possèdent la signification indiquée ci-dessus, avec un dérivé du phosgène
puis
b) on fait réagir l'ester d'acide chlorocarbonique formé dans l'étape a) avec des nucléosides de formule générale (III) 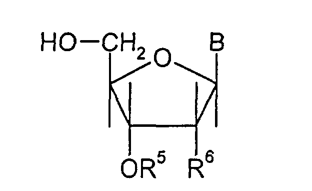 où R⁵, R⁶ et B possèdent la signification indiquée ci-dessus, puis, éventuellement
c) on introduit dans la position 3' des dérivés de nucléosides avec R⁵ = H le groupe phosphitamide 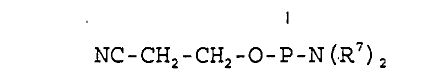
ou 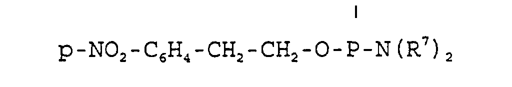 selon des méthodes connues.

15. Procédé selon la revendication 14 **caractérisé en ce que** l'on conduit l'étape a) dans un solvant organique non polaire à des températures situées entre -20 et +25°C.

16. Procédé selon les revendications 14 et 15 **caractérisé en ce que**, dans l'étape a), on utilise le dérivé du phosgène en un excès de deux à cinq fois par rapport au composant alcool.

17. Procédé selon les revendications 14 à 16 **caractérisé en ce que**, dans l'étape a), on utilise le toluène ou le THF comme solvant organique non polaire.

18. Procédé selon les revendications 14 à 17 **caractérisé en ce que** la concentration du composant alcool dans l'étape a) est de 0,1 à 10,0 mmol pour 10 ml de solvant.

19. Procédé selon les revendications 14 à 18 **caractérisé en ce que** l'on réalise l'étape b) dans un mélange de solvants consistant en dichlorométhane et en un solvant organique polaire éventuellement en présence d'une base à des températures sit +25°C.

20. Procédé selon les revendications 14 à 19 **caractérisé en ce que**, dans l'étape b), on utilise le DMF ou la pyridine comme solvant organique polaire.

21. Procédé selon les revendications 14 à 20 **caractérisé en ce que**, dans l'étape b), on utilise un mélange de solvants consistant en dichlorométhane et en DMF ainsi qu'une base choisie dans le groupe de la pyridine, de la triéthylamine et de l'éthyldiisopropylamine.

22. Procédé selon les revendications 20 à 21 **caractérisé en ce que** le rapport de mélange du dichlorométhane à la pyridine ou au DMF est 1:1 à 3:1.

23. Procédé selon les revendications 14 à 22 **caractérisé en ce que** le rapport molaire du nucléoside à l'ester d'acide chlorocarbonique est 1:1 à 1:2.

24. Procédé selon les revendications 14 à 23 **caractérisé en ce que**, dans l'étape b), on dispose au préalable le nucléoside dissous dans la pyridine ou le DMF/base et on ajoute goutte à goutte une solution de l'ester d'acide chlorocarbonique dans le dichlorométhane à la température de réaction correspondante.

25. Procédé selon les revendications 14 à 24 **caractérisé en ce que** la concentration du nucléoside dans le mélange de solvants dans l'étape b) est de 0,1 à 3,0 mmol pour 10 ml de solvant.

26. Procédé selon les revendications 14 à 25 **caractérisé en ce que** l'on réalise l'introduction du groupe phosphitamide (étape c) par réaction des dérivés de nucléosides (avec R⁵ = H) avec les phosphines correspondantes en présence de 1H-tétrazole comme activateur dans un mélange de solvants consistant en dichlorométhane et en acétonitrile à des températures situées entre 0 et 25°C.

27. Utilisation d'un dérivé de nucléoside de formule générale (I) selon la revendication 1 pour la synthèse d'oligonucléotides commandée par la lumière.

28. Utilisation selon la revendication 27 **caractérisée en ce que** la synthèse d'oligonucléotides commandée par la lumière a lieu sur un support solide.

29. Utilisation selon la revendication 27 ou 28 **caractérisée en ce que** le dérivé de nucléoside est défini comme dans une ou plusieurs des revendications 2 à 13.
